# EUROPÄISCHE PATENTSCHRIFT

(11) **EP 1 807 702 B1**
(45) Veröffentlichungstag und Bekanntmachung des Hinweises auf die Patenterteilung: **05.08.2009**
(21) Anmeldenummer: 05798099.7
(22) Anmeldetag: 25.10.2005
(51) Int. Cl.: G01N 33/74, G01N 33/558, G01N 33/68

(54) **P1GF UND FLT-1 ALS PROGNOSTISCHE PARAMETER BEI KARDIOVASKULÄREN ERKRANKUNGEN**
P1GF AND FLT-1 AS PROGNOSTIC PARAMETERS FOR CARDIOVASCULAR DISEASES
P1GF ET FLT-1 EN TANT QUE PARAMETRES PRONOSTIQUES DANS LE CADRE DE TROUBLES CARDIO-VASCULAIRES

(30) Priorität: 25.10.2004 DE 102004051847
(43) Veröffentlichungstag der Anmeldung: 18.07.2007
(73) Patentinhaber: Siemens Healthcare Diagnostics Products GmbH, 35041 Marburg (DE)
(72) Erfinder: ZEIHER, Andreas, M., 60594 Frankfurt (DE); HEESCHEN, Christopher, 81247 München (DE); DIMMELER, Stefanie, 60594 Frankfurt (DE)
(74) Vertreter: Schohe, Stefan
(86) Internationale Anmeldenummer: PCT/EP2005/011443
(87) Internationale Veröffentlichungsnummer: WO 2006/045593

(56) Entgegenhaltungen:
- WO-A-98/28006
- WO-A-03/000183
- US-A1- 2004 126 828
- LUTTUN A ET AL: "Revascularization of ischemic tissues by PlGF treatment, and inhibition of tumor angiogenesis, arthritis and atherosclerosis by anti-Flt1" NATURE MEDICINE, NATURE PUBLISHING GROUP, NEW YORK, NY, US, Bd. 8, Nr. 8, August 2002 (2002-08), Seiten 831-840, XP002258708 ISSN: 1078-8956
- MAYNARD SHARON E ET AL: "Excess placental soluble fms-like tyrosine kinase 1 (sFlt1) may contribute to endothelial dysfunction, hypertension, and proteinuria in preeclampsia" JOURNAL OF CLINICAL INVESTIGATION, NEW YORK, NY, US, Bd. 111, Nr. 5, März 2003 (2003-03), Seiten 649-658, XP002314744 ISSN: 0021-9738
- LUTTUN AERNOUT ET AL: "Placental growth factor (PlGF) and its receptor Flt-1 (VEGFR-1): novel therapeutic targets for angiogenic disorders." ANNALS OF THE NEW YORK ACADEMY OF SCIENCES. DEC 2002, Bd. 979, Dezember 2002 (2002-12), Seiten 80-93, XP002373381 ISSN: 0077-8923

## Beschreibung

Die vorliegende Erfindung betrifft eine Verwendung eines Verfahrens *ex vivo,* das die Bestimmung von PIGF und sFlt-1 in einer Probe umfasst, zur Diagnose, Risikostratifizierung und/oder Überwachung einer vaskulären Erkrankung mit atherosklerotischer Ätiologie und/oder zur Abschätzung der Wahrscheinlichkeit, eine derartige Erkrankung zu entwickeln. Die vorliegende Erfindung betrifft auch das verwendete Verfahren. Weiterhin betrifft die Erfindung ein diagnostisches Kit und seine Verwendung.

### Stand der Technik

In allen Stadien einer Atherosklerose, d.h. von der Entstehung früher atheroskleorischer Läsionen über deren Progression bis hin zu Erosion bzw. Ruptur der Läsionen mit entsprechenden thrombotischen Komplikationen, spielen entzündliche Vorgänge eine fundamentale Rolle. Überzeugende Befunde weisen darauf hin, dass an der Destabilisierung einer atherosklerotischen Läsion mit der Folge eines akuten Koronarsyndroms ebenfalls entzündliche Mechanismen beteiligt sind (1, 2).

Aufgrund des Zusammenhangs zwischen Entzündung und Atherosklerose werden etablierte Entzündungsmarker, die ins zirkulierende Blut freigesetzt werden, auch zur Risikostratifizierung von Patienten mit einer akuten koronaren Herzerkrankung herangezogen. Im Gegensatz etwa zu den Troponinen, die Marker einer Zellnekrose darstellen und damit den Endzustand eines Myokardinfarkts anzeigen, können Entzündungsmarker ein entsprechendes Risiko noch vor dem Auftreten einer Myokardschädigung anzeigen, da sie die Entzündungsprozesse widerspiegeln, die einem akuten Koronarsyndrom zugrunde liegen.

Von den etablierten Entzündungsmarkern haben das C-reaktive Protein (CRP, hier auch hsCRP, d.h. "hochsensitives CRP", genannt) und Fibrinogen die größte Aufmerksamkeit auf sich gezogen, und der prognostische Wert dieser Marker in Bezug auf Mortalität und ischämische Ereignisse wurde eindeutig nachgewiesen (22-24). In retrospektiven Untersuchungen wurde gezeigt, daß CRP und Fibrinogen eine eigene Bedeutung als prognostische Parameter haben und sie deshalb als zusätzliche Marker zu Troponin T in Betracht kommen (14, 25, 26). Für CRP ist gezeigt worden, dass dieser Marker für die Langzeitprognose bei einer koronaren Herzerkrankung nützlich ist, sein Wert als Marker in der Akutphase, also im Rahmen eines akuten Koronarsyndroms, wird jedoch widersprüchlich beurteilt (14, 27).

Ein erstes Ergebnis der CAPTURE-Studie war, dass in der frühen Phase von 72 Stunden nach Beginn der Symptome eines akuten Koronarsyndroms nur Troponin T eine zuverlässige Vorhersage ermöglichte, im Gegensatz dazu jedoch sowohl Troponin T als auch CRP unabhängige prognostische Parameter eines Risikos in den nachfolgenden sechs Monaten waren (14). Vergleichbare Ergebnisse wurden auch für die GUSTO IV-ACS-Studie berichtet (27). Die genaue Quelle der erhöhten CRP-Spiegel bei Patienten mit instabiler Koronarerkrankung bleibt weiterhin unklar. Im Zusammenhang mit der Annahme, dass eine Schädigung des Myokards ebenfalls ein bedeutender Entzündungsstimulus ist, muss zur Kenntnis genommen werden, dass in einer neueren kombinierten Analyse von FRISC-II und GUSTO-IV ein CRP-Anstieg über einen Zeitraum von bis zu 120 Stunden nur bei Patienten mit erhöhten Troponin-Spiegeln gefunden wurde (27). Ähnlich waren bei Troponin-positiven Patienten der CAPTURE-Studie die CRP-Spiegel signifikant höher (14), was darauf hindeutet, dass ein akuter entzündlicher Prozess, der auf eine Schädigung des Myokards zurückgeht, eine chronische Entzündung in der Gefäßwand überlagert, so dass der chronische Entzündungsprozess im Rahmen eines akuten Koronarsyndroms mittels CRP kaum abgeschätzt werden kann. Weiterhin ist anzumerken, dass pro-inflammatorische Zytokine auch von Fettgewebe, Gewebsmakrophagen und dem verletzten Myokard freigesetzt werden.

Erst kürzlich wurde gezeigt, dass der plazentale Wachstumsfaktor (*placental growth factor,* PlGF), ein Mitglied der VEGF-Familie (*vascular endothelial growth factor,* VEGF), in frühen und fortgeschrittenen atherosklerotischen Läsionen verstärkt exprimiert ist (3). Ursprünglich identifiziert in der Plazenta (4), stimuliert PlGF das Wachstum glatter Gefäßmuskelzellen, veranlasst Makrophagen in atherosklerotische Läsionen einzuwandern, fördert die Produktion verschiedener Entzündungsmediatoren in Makrophagen (Tumornekrosefaktor-α (TNF-α), monozytisches chemotaktisches Protein-1 (MCP-1), Proteasen) und stimuliert die pathologische Angiogenese in der Gefäßwand (3, 5). Eine Hemmung der Wirkungen von PlGF durch Blockieren seines membranständigen Rezeptors Flt-1 (*Fms-like tyrosine kinase 1*) in einem Tiermodell für Atherosklerose unterdrückte das Wachstum atherosklerotischer Plaques und zeigte günstige Effekte auf deren Stabilität, indem die Makrophageninfiltration gehemmt wurde (3, 6). Bei Patienten mit akuten koronaren Herzerkrankungen wurde kürzlich gezeigt, dass die PlGF-Konzentrationen im Plasma erhöht sind, und dass die systemische PlGF-Konzentration einen leistungsfähigen klinischen Marker für eine Gefäßentzündung und die entsprechenden, für die Patienten nachteiligen Folgen darstellt (7).

Flt-1 bindet außer PlGF auch den verwandten Faktor VEGF (8) und tritt in zwei Formen auf: einerseits als membrangebundene Rezeptortyrosinkinase (Flt-1), welche die angiogenen Signale ins Zellinnere überträgt, und andererseits als eine lösliche Ektodomäne (*soluble Flt-1,* sFlt-1), deren Aufgabe es ist, die freien Faktoren PlGF oder VEGF in der Zirkulation abzufangen (6). Da der löslichen Form von Flt-1 eine zytosolische Domäne fehlt, ist die Funktion von sFlt-1 darauf beschränkt, die Menge an zirkulierendem PLGF oder VEGF, die als freie Faktoren zur Aktivierung der membrangebundenen Rezeptoren Flt-1 und Flk-1 (fötale Leberkinase-1) verfügbar sind, zu regulieren (9). Während eines akuten koronaren Herzsydroms konnten erhöhte Konzentrationen des löslichen PlGF-Rezeptors sFlt-1 nachgewiesen werden (10).

Die Patentanmeldung WO 2004/046722 (Dimmeler et al.) offenbart ein Verfahren zur Analyse von Proben im Zusammenhang mit akuten kardiovaskulären Erkrankungen, wobei das Verfahren die Messung der Konzentration eines Markers, z.B. PIGF, und gegebenenfalls eines weiteren Markers, z.B. von VEGF oder einem weiteren Entzündungsmarker, umfasst.

Aus der Patentanmeldung US 2004/126828 (Karumanchi et al.) ist ein Verfahren zur Diagnose von Präeklampsie oder Eklampsie bekannt, welches die Messung der Konzentration von sFlt-1, VEGF oder PIGF umfasst. sFlt-1 ist als ein möglicher Kandidat für einen Präeklampsie-Faktor beschrieben worden (17), da nicht nur die Plazenta von Schwangeren mit Präeklampsie erhöhte Mengen an sFlt-1 produziert, sondern erhöhte sFlt-1-Spiegel auf die spätere Entwicklung einer Päeklmapsie hinweisen (18). In der US 2004/126828 wird eine erhöhte Konzentration von sFlt-1, insbesondere Serumspiegel > 2.000 ng/l, und eine erniedrigte Konzentration von VEGF dabei als positive diagnostische Indikatoren einer Präeklampsie angesehen. Werden die für die drei Marker erhaltenen Ergebnisse miteinander in Beziehung gesetzt werden, um einen so genannten "angiogenen Index" zu ermitteln, ist eine bestehende Präeklampsie oder ein erhebliches Risiko, eine solche zu entwickeln, dann festzustellen, wenn der angiogene Index, bestimmt nach der Formel [sFlt-1/VEGF + P1GF], > 20 beträgt, d.h. wenn die Konzentration von s-Flt-1 mehr als 20-mal so hoch ist wie die von VEGF und PIGF zusammen.

Die Patentanmeldung WO 2005/031364 (Thadhani und Karumanchi) beschreibt ein Verfahren zur Diagnose oder Prognose einer Gestose, wie etwa Präemklampsie, das die Messung von Sexualhormon-bindendem Globulin (SHBG) und PIGF, und in einer besonderen Ausführung sFlt-1, umfasst.

Der Patentanmeldung WO 2005/017192 (Thadhani et al.*)* ist zu entnehmen, dass die bei einer Präeklampsie gemessenen Serumspiegel von PIGF deutlich niedriger (ca. 6-fach) und die von sFlt-1 höher (ca. 2-fach) waren als die in Kontrollproben gemessenen Werte. Dementsprechend betrug das Verhältnis von sFlt-1 zu PIGF im Fall einer Präeklampsie das 15-fache von dem für eine Kontrollprobe ermittelten Faktor.

Die Patentanmeldung WO 98/28006 offenbart ein Verfahren zur Diagnose von Schwangerschaftshypertonie (Präeklampsie), bei dem die Menge an PIGF, VEGF und die an einem löslichen VEGF-Rezeptor, wie etwa sFLT-1, in einer Probe bestimmt wird.

Dem Krankheitsbild Präeklampsie bzw. Eklampsie liegt jedoch eine völlig andere Ätiologie zugrunde als etwa der koronaren Herzerkrankung, insbesondere sind diese Gestosen nicht das Ergebnis einer atherosklerotischen Erkrankung. Insofern sind die im Stand der Technik offenbarten Verfahren nicht auf vaskuläre Erkrankungen mit atherosklerotischer Ätiologie, wie sie etwa eine koronare Herzerkrankung darstellt, übertragbar.

Ausgehend vom Stand der Technik war es deshalb die Aufgabe der Erfindung, ein Verfahren verfügbar zu machen, das auf der Grundlage der Messung von Biomarkern eine Abschätzung der Wahrscheinlichkeit für eine Entwicklung, eine Diagnose, eine Risikostratifizierung und/oder eine Überwachung einer vaskulären Erkrankung mit atherosklerotischer Ätiologie ermöglicht.

### Kurze Darstellung der Erfindung

Gelöst wird die Aufgabe der vorliegenden Erfindung durch Bereitstellen der erfindungsgemäßen Verfahren, Verwendungen und Vorrichtungen gemäß den Patentansprüchen.

Eine Lösung der Aufgabe ist ein Verfahren zur Diagnose, Risikostratifizierung und/oder Überwachung einer vaskulären Erkrankung mit atherosklerotischer Ätiologie und/oder zur Abschätzung der Wahrscheinlichkeit, eine derartige Erkrankung zu entwickeln, wobei das Verfahren folgende Schritte umfasst:
(a) Verfügbarmachen einer zu untersuchenden Probe eines Patienten;
(b) Quantifizieren von PIGF in der Probe;
(c) Quantifizieren von sFlt-1 in der Probe.
   Dieses Verfahren kann auch folgenden Schritt umfassen:
(d) Bilden eines Verhältnisses zwischen dem in (b) ermittelten Wert von PIGF und dem in (c) ermittelten Wert von sFlt-1.

Die "Bildung des Verhältnisses zwischen dem in (b) ermittelten Wert von PIGF und dem in (c) ermittelten Wert von sFlt-1" umfasst sowohl das Berechnen des Quotienten "in (b) ermittelter Wert von PlGF/ in (c) ermittelter Wert" als auch andere Möglichkeiten den in (b) ermittelten Wert von PlGF mit dem in (c) ermittelten Wert von sFlt-1 in Beziehung zu setzen.

Eine Lösung der Aufgabe ist somit auch ein Verfahren zur Diagnose, Risikostratifizierung und/oder Überwachung einer vaskulären Erkrankung mit atherosklerotischer Ätiologie und/oder zur Abschätzung der Wahrscheinlichkeit, eine derartige Erkrankung zu entwickeln, wobei das Verfahren folgende Schritte umfasst:
(a) Verfügbarmachen einer zu untersuchenden Probe eines Patienten;
(b) Quantifizieren von PlGF in der Probe;
(c) Quantifizieren von sFlt-1 in der Probe; und
(d) Vergleich der in (b) und (c) ermittelten Werte von PIGF und sFlt-1 mit jeweils einem Referenzwert und/oder jeweils einem in einer Referenzprobe ermittelten Wert.

Das Verfahren kann auch folgende Schritte umfassen:
(a) Verfügbarmachen einer zu untersuchenden Probe eines Patienten;
(b) Quantifizieren von PlGF in der Probe;
(c) Quantifizieren von sFlt-1 in der Probe.
(d') Bilden eines Verhältnisses zwischen dem in (b) ermittelten Wert von PlGF und dem in (c) ermittelten Wert von sFlt-1, vorzugsweise die Berechnung des Quotients PlGF/sFlt-1 und/oder des Quotients sFlT-1/PlGF; und
(e') Vergleich des in (d') ermittelten Werts mit einem Referenzwert und/oder einem in einer Referenzprobe ermittelten Wert.

Das Verfahren kann auch folgende Schritte umfassen:
(a) Verfügbarmachen einer zu untersuchenden Probe eines Patienten;
(b) Quantifizieren von PlGF in der Probe;
(c) Quantifizieren von sFlt-1 in der Probe.
(d) Vergleich der in (b) und (c) ermittelten Werte von PlGF und sFlt-1 mit jeweils einem Referenzwert und/oder jeweils einem in einer Referenzprobe ermittelten Wert.
(d') Bilden eines Verhältnisses zwischen dem in (b) ermittelten Wert von PIGF und dem in (c) ermittelten Wert von sFlt-1, vorzugsweise die Berechnung des Quotients PIGF/sFlt- und/oder des Quotients sFlT-1/PlGF; und
(e') Vergleich des in (d') ermittelten Werts mit einem Referenzwert und/oder einem in einer Referenzprobe ermittelten Wert.

Die Schritte (b) und (c) können nacheinander in dieser Reihenfolge, in umgekehrter Reihenfolge oder zeitgleich durchgeführt werden.

In Schritt (d) wird der in (b) ermittelte Wert mit einem Referenzwert für PIGF und/oder einem in einer Referenzprobe ermittelten Werte für PIGF verglichen, und der in (c) ermittelte Wert wird mit einem Referenzwert für sFlt-1 und/oder einem in einer Referenzprobe ermittelten Wert für sFlt-1 verglichen. In Schritt (e') wird der in (d') ermittelte Wert (insbesondere der Quotient PIGF/sFlt-1 und/oder der Quotient sFlT-1/PIGF) mit einem Referenzwert für das Verhältnis von PIGF und sFLT-1 und/oder einem in einer Referenzprobe ermittelten Wert für dieses Verhältnis verglichen.

Bei der vorliegenden Erfindung handelt es sich um ein Verfahren, das *ex vivo* durchgeführt wird, d.h. ein in vitro-Verfahren.

Die Bezeichnung "vaskuläre Erkrankung mit atherosklerotischer Ätiologie" schließt die Erkrankung Präeklampsie bzw. Eklampsie aus. Der Betriff "atherosklerotisch" betrifft sowohl eine stabile als auch eine instabile Atherosklerose.

Der Begriff "Verfügbarmachen" einer zu untersuchenden Probe, wie hier verwendet, ist mit dem "Bereitstellen" einer Probe gleichzusetzen. Hiermit ist gemeint, dass eine vorhandene, zu untersuchende Probe für die in vitro Messverfahren bereitgestellt wird, z.B. in das Messinstrument eingeführt wird. Die zu untersuchende Probe, vorzugsweise Blutplasma oder -serum, und/oder die Referenzprobe können vorbehandelt sein, z.B. indem peripherem Blut ein Gerinnungshemmer, insbesondere EDTA, Heparin oder Citrat, zugesetzt wurde. Von dem Begriff "Verfügbarmachen" nicht umfasst ist die Probenentnahme per se, z.B. die invasive Entnahme einer Probe eines Patienten, beispielsweise durch Punktieren, oder eine nicht-invasive Probengewinnung, wie etwa die Gewinnung einer Urinprobe.

In einer bevorzugten Ausführung der vorliegenden Erfindung ist der Patient ein Säugetier, besonders bevorzugt ein Mensch. Der Begriff "Patient" bezeichnet insbesondere eine von einem Arzt oder einem Angehörigen anderer Heilberufe behandelte Person und umfasst sowohl Kranke als auch Gesunde oder scheinbar Gesunde.

Das "Quantifizieren" von PIGF und/oder sFlt-1 kann in einem Bestimmen einer Konzentration, beispielsweise einer Proteinkonzentration bestehen. Außer in einer Konzentrationsbestimmung, z.B. in Blutplasma oder -serum, kann das Quantifizieren auch in einer Bestimmung der Anzahl der Moleküle, z.B. in einem histologischen Gewebeschnitt, bestehen. Das "Quantifizieren" schließt auch semiquantitative Bestimmungsmethoden mit ein, die nur die ungefähre Menge oder Konzentration von PIGF und/oder sFlt-1 in der Probe erfassen oder nur zu einer relativen Mengen- oder Konzentrationsangabe dienen können oder nur anzeigen, ob die Menge oder Konzentration von PIGF und/oder sFlt-1 in der Probe jeweils unterhalb oder oberhalb eines bestimmten oder mehrerer bestimmter Referenzwerte liegt.

Der Begriff "Referenzwert" kann ein vorgegebener Wert sein oder ein in einer Referenzprobe ermittelter Wert sein. Eine "Referenzprobe" kann beispielsweise von Gesunden oder von Patienten mit oder ohne stabile bzw. instabile Atherosklerose stammen, vorzugsweise von Patienten mit akutem Koronarsyndrom, ganz besonders bevorzugt von Patienten mit instabiler Angina pectoris oder einem akutem Myokardinfarkt. Es kann sich auch um eine Probe handeln, der PIGF und sFlt-1 in einem Verhältnis zugesetzt wurde, das bei Gesunden oder bei Patienten mit einer vaskulären Erkrankung mit atherosklerotischer Ätiologie früher gemessen wurde. Üblicherweise werden verschiedene Referenzproben eingesetzt, welche die verschiedenen möglichen Prognosen, z.B. "nachteiliges Ereignis unwahrscheinlich" bis "nachteiliges Ereignis hoch wahrscheinlich", angeben. Das Bereitstellen von Referenzproben erfolgt vorzugsweise auf die gleiche Weise, wie das Bereitstellen der zu untersuchenden Probe. Anstatt des Einsatzes von Referenzproben können auch festgelegte Referenzwerte, die beispielsweise aus einer Tabelle abzulesen sind, verwendet werden. Derartige Referenzwerte können beispielsweise verschiedene Bereiche festlegen, welche die Wahrscheinlichkeit eines Ereignisses angeben.

Vorzugsweise ist ein Referenzwert und/oder ein in einer Referenzprobe ermittelter Wert ein "*Cut-off-*Wert" oder "Grenzwert", d.h. ein Wert, der eine Grenze angibt. Bei einem Vergleich eines in einer Probe ermittelten Werts mit einem *Cut-off*-Wert ergibt sich, dass ein Messwert oberhalb der Grenze zu einer anderen Beurteilung führt als ein Messwert unterhalb der Grenze. In Bezug auf die vorliegende Erfindung wäre beispielsweise die PIGF-Konzentration als ein geeigneter PIGF-*Cut-off*-Wert anzusehen, welche die beiden oberen Tertilen eines geeigneten Referenzkollektivs von der unteren Tertile trennt. Ein anderer geeigneter PIGF-*Cut-off-*Wert ist die PIGF-Konzentration, welche die oberste Tertile eines geeigneten Referenzkollektivs von der mittleren Tertile trennt. Ein geeigneter sFlt-1-*Cutt-off*-Wert ist beispielsweise die sFlt-1-Konzentration, welche die mittlere Tertile eines geeigneten Referenzkollektivs von der unteren Tertile trennt. Neben der Ermittlung des jeweiligen *Cut-off*-Werts über Tertilen können auch mittels ROC- (*receiver operating curve*) Kurven und anderen gängigen Methoden (s.a. "A. PATIENTEN UND METHODEN, 4. Statistische Verfahren") für die Erfindung geeignete *Cut-off-*Werte bestimmt werden. So kann die anhand eines geeigneten Referenzkollektivs ermittelte mediane PIGF- bzw. sFlt-1-Konzentration als PIGF-*Cut-off`-* bzw. sFlt-1-*Cut-off-*Wert dienen. In Bezug auf die vorliegende Erfindung würde das Überschreiten eines geeigneten PIGF-*Cut-off*-Wertes und das gleichzeitige Unterschreiten eines geeigneten sFlt-1-*Cut-off-*Wertes anzeigen, dass eine erhöhte Wahrscheinlichkeit besteht, dass der betroffene Patient einen Myokardinfarkt oder Schlaganfall erleiden wird und/oder an einer vaskulären Erkrankung mit atherosklerotischer Ätiologie sterben wird.

Eine besonders vorteilhaftes erfindungsgemäßes Verfahren ist die Verwendung eines Verfahrens, das folgende Schritte umfasst:
(a) Verfügbarmachen einer zu untersuchenden Probe eines Patienten;
(b) Quantifizieren von PIGF in der Probe;
(c) Quantifizieren von sFlt-1 in der Probe,
(d) Vergleich der in (b) und (c) ermittelten Werte von PIGF und sFlt-1 mit jeweils einem Referenzwert und/oder jeweils einem in einer Referenzprobe ermittelten Wert.
   zur Diagnose, Risikostratifizierung und/oder Überwachung einer vaskulären Erkrankung mit atherosklerotischer Ätiologie bei einem Patienten und/oder zur Abschätzung der Wahrscheinlichkeit für einen Patienten, eine derartige Erkrankung zu entwickeln.

Die Verwendung des Verfahrens kann auch folgende Schritte umfassen:
(a) Verfügbarmachen einer zu untersuchenden Probe eines Patienten;
(b) Quantifizieren von PlGF in der Probe;
(c) Quantifizieren von sFlt-1 in der Probe.
(d') Bilden eines Verhältnisses zwischen dem in (b) ermittelten Wert von PIGF und dem in (c) ermittelten Wert von sFlt-1, vorzugsweise die Berechnung des Quotients PlGF/sFlt-1 und/oder des Quotients sFIT-1/PIGF; und
(e') Vergleich des in (d') ermittelten Werts mit einem Referenzwert und/oder einem in einer Referenzprobe ermittelten Wert.

Die Verwendung des Verfahrens kann auch folgende Schritte umfassen:
(a) Verfügbarmachen einer zu untersuchenden Probe eines Patienten;
(b) Quantifizieren von PIGF in der Probe;
(c) Quantifizieren von sFlt-1 in der Probe.
(d) Vergleich der in (b) und (c) ermittelten Werte von PlGF und sFlt-1 mit jeweils einem Referenzwert und/oder jeweils einem in einer Referenzprobe ermittelten Wert.
(d') Bilden eines Verhältnisses zwischen dem in (b) ermittelten Wert von PIGF und dem in (c) ermittelten Wert von sFlt-1, vorzugsweise die Berechnung des Quotients PIGF/sFlt- und/oder des Quotients sFIT-1/PIGF; und
(e') Vergleich des in (d') ermittelten Werts mit einem Referenzwert und/oder einem in einer Referenzprobe ermittelten Wert.

Die Schritte (b) und (c) können nacheinander in dieser Reihenfolge, in umgekehrter Reihenfolge oder zeitgleich durchgeführt werden.

Die Aufgabe der Erfindung wird weiterhin gelöst durch ein diagnostisches Kit, umfassend mindestens ein Mittel zum Quantifizieren von PIGF und mindestens ein Mittel zum Quantifizieren von sFlt-1 in einer zu untersuchen Probe, wobei das Kit auch aus separaten Packungen bestehen kann und wobei das Kit weiterhin ein Mittel zur Information (z.B. Packungsbeilage) umfasst, wonach (i) ein Verhältnis von [PIGF = hoch : sFlt-1 = niedrig] und/oder (ii) eine PIGF-Konzentration, welche in den oberen beiden Tertilen eines Referenzkollektivs liegt, und eine sFlt-1-Konzentration, welche im untersten Tertil des Referenzkollektivs liegt, und/oder (iii) ein PIGF-Wert oberhalb des PIGF-Referenzwerts und ein sFlt-1-Wert unterhalb des sFlt-1-Referenzwerts beispielsweise eine erhöhte Wahrscheinlichkeit für ein nachteiliges Ereignis wie z.B. Tod, nicht-tödlicher Myokardinfarkt und/oder Schlaganfall anzeigt.

Die Aufgabe der vorliegenden Erfindung wird auch gelöst durch eine Verwendung des erfindungsgemäßen Kits zur Diagnose, Risikostratifizierung und/oder Überwachung einer vaskulären Erkrankung mit atherosklerotischer Ätiologie und/oder zur Abschätzung der Wahrscheinlichkeit, eine derartige Erkrankung zu entwickeln.

Die Aufgabe der vorliegenden Erfindung wird auch gelöst durch eine Verwendung des erfindungsgemäßen Kits zur Durchführung des erfindungsgemäßen Verfahrens.

Im folgenden sollen der kurzen Darstellung nähere Erläuterungen und weitere Ausführungen der Erfindung hinzugefügt werden.

In einer Ausführung des Verfahrens und der Verwendung des Verfahrens ist die vaskuläre Erkrankung ausgewählt aus der Gruppe bestehend aus einer organbezogenen Gefäßerkrankung (insbesondere einer koronaren Herzerkrankung oder einer cerebrovaskulären Erkrankung) und/oder einer peripheren Gefäßerkrankung (insbesondere eine arterielle oder venöse Verschlusskrankheit). In einer weiteren Ausführung des Verfahrens ist die vaskuläre Erkrankung ein akutes Koronarsyndrom, vorzugsweise eine instabile Angina pectoris oder ein akuter Myokardinfarkt. In einer bevorzugten Ausführung des Verfahrens ist die koronare Herzerkrankung ein akutes Koronarsyndrom. In einer bevorzugten Ausführung des Verfahren werden nur Proben von Patienten eingesetzt, die an einer vaskulären Erkrankung, wie oben näher bezeichnet, insbesondere an einem akuten Koronarsyndrom (z.B. an einem Myokardinfarkt) leiden oder im Verdacht stehen, eine solche Erkrankung zu haben oder zukünftig zu entwickeln. Die Probe kann aber auch von "zufällig" ausgewählten Patienten stammen, beispielsweise im Rahmen eines *Screenings* oder einer Vorsorgeuntersuchung. Vorzugsweise wird das erfindungsgemäße Verfahren bei einem akuten Koronarsyndrom, wie etwa Angina pectoris und/oder aktutem Myokardinfarkt verwendet.

Bei der zu untersuchenden Probe handelt es sich vorzugsweise um peripheres Blut oder eine Fraktion davon, ganz besonders bevorzugt ist die Fraktion Blutplasma (Plasma) oder Blutserum (Serum). In einer anderen Ausführungsform der Erfindung werden auch andere Körperflüssigkeiten (z.B. Urin oder Liquor) sowie Gewebeproben, Suspensionen von Gewebezellen, Gewebehomogenate oder Gewebeschnitte als zu untersuchende Probe eingesetzt. Unter einer "Probe" ist im Sinne der Erfindung ein Material zu verstehen, das die nachzuweisende Substanzen PlGF und sFlt-1 vermutlich enthält. Gegebenenfalls müssen die Proben vorbehandelt werden, um die nachzuweisende Substanzen für das jeweilige Nachweisverfahren zugänglich zu machen oder um störende Probenbestandteile zu entfernen. Eine solche Vorbehandlung von Proben mag die Abtrennung und/oder Lyse von Zellen beinhalten, das Präzipitieren, die Hydrolyse oder die Denaturierung von Probenbestandteilen, wie z.B. Proteinen, die Zentrifugation von Proben, das Behandeln der Probe mit organischen Lösungsmitteln, wie z.B. durch Alkohole, insbesondere Methanol, oder das Behandeln der Probe mit Detergenzien.

In einer bevorzugten Ausführung der vorliegenden Erfindung ist der Patient ein Säugetier, besonders bevorzugt ein Mensch und ganz besonders bevorzugt ein Mensch mit einer vaskulären Erkrankung, wie oben näher bezeichnet, vorzugsweise mit einem akuten Koronarsyndrom, wie z.B. einem Myokardinfarkt. In einer ganz besonders bevorzugten Ausführung des erfindungsgemäßen Verfahrens werden nur Proben von Patienten gemessen, bei denen das Vorliegen einer Schwangerschaft ausgeschlossen werden kann oder mit mindestens einer überwiegenden Wahrscheinlichkeit ausgeschlossen wurde.

In einer bevorzugten Ausführung wird das erfindungsgemäße Verfahren zur Risikostratifizierung einer vaskulären Erkrankung mit atherosklerotischer Ätiologie verwendet bzw. umfasst das Verfahren die Durchführung einer Risikostratifizierung. Die Risikostratifizierung umfasst die Bestimmung einer Wahrscheinlichkeit, dass ein Patient ein nachteiliges Ereignis erleidet, wie z.B. Tod, nicht-tödlicher Myokardinfarkt oder Schlaganfall. Das nachteilige Ereignis kann auch eine nachteilige Folgeerscheinung sein, die beispielsweise darin besteht, einen weiteren nicht-tödlichen Myokardinfarkt zu erleiden oder nach einem sich erstmals ereignenden nicht-tödlichen Myokardinfarkt einen Schlaganfall zu bekommen oder zu versterben.

Die erfindungsgemäßen Verfahren zeigen (i) bei einem PIGF-Wert oberhalb des PIGF-Referenzwerts und einem sFlt-1-Wert unterhalb des sFlt-1-Referenzwerts und/oder (ii) bei einer PlGF-Konzentration, welche in den oberen beiden Tertilen eines Referenzkollektivs liegt, und bei einer sFlt-1-Konzentration, welche im untersten Tertil des Referenzkollektivs liegt, und/oder (iii) bei einem Verhältnis von [PlGF = hoch : sFlt-1 = niedrig] eine erhöhte Wahrscheinlichkeit für ein nachteiliges Ereignis an.

Der Begriff "Referenzkollektiv" bezeichnet in der Regel eine Gruppe von Referenzindividuen, die vorzugsweise zufällig aus der Gesamtheit einer Population ausgewählt wurden, die bestimmte Selektionskriterien erfüllt. Aus praktischen Gründen wird oft statt einer rein zufälligen Auswahl von Individuen aus einer Gesamtheit einer Population bzw. einem Gesamtkollektiv, ein Referenzkollektiv anhand praktischer Überlegungen aus geeigneten, zur Verfugung stehenden Individuen erstellt. Möglichst klar definierte Selektionskriterien sind beispielsweise definierte und typische Erkrankungen, beispielsweise eine instabilen Angina pectoris, ein akuter Myokardinfarkt, etc. Daneben sind Referenzkollektive von gesunden Individuen, undifferenzierten und hospitalisierten Individuen, etc. relevant, um populationsbasierte Referenzwerte für die entsprechenden Kollektive zu ermitteln. Das im Hinblick auf diese Erfindung bevorzugte Referenzkollektiv besteht aus einer für statische Zwecke hinreichend großen Anzahl an Individuen, die an einer vaskulären Erkrankung mit atherosklerotischer Ätiologie leiden, insbesondere an einem akutern Koronarsyndrom, wie z.B. einer instabilen Angina pectoris oder einem akuten Myokardinfarkt. Referenzkollektive können auch aus Patienten rekrutiert werden, die eine erhöhte oder erniedrigte Ereignisrate aufweisen.

Neben Referenzwerten, die auf einem Referenzkollektiv basieren, können auch "Subjekt-basierte Referenzwerte" zum Einsatz kommen. Subjekt-basierte Referenzwerte sind schon vorhandene Werte (z.B. Konzentration eines Biomarkers wie PIGF oder sFlt-1) eines einzigen Individuums, die zu einem Zeitpunkt ermittelt wurden, als sich das Individuum in einem definierten Gesundheits- bzw. Krankheitszustand befand.

In einer bevorzugten Ausführung des erfindungsgemäßen Verfahrens wird ein PIGF*-Cut-off-*Wert ≥ 17,7 ng/l als Referenzwert verwendet. In einer anderen bevorzugten Ausführung des erfindungsgemäßen Verfahrens wird ein PlGF-*Cut-off*-Wert ≥ 23,3 ng/l als Referenzwert verwendet. Auch ein PIGF-*Cut-off*-Wert ≥ 15,6 ng/l kann verwendet werden. Bevorzugt wird ein PIGF-*Cut-off* im Bereich von 15,6-23,3 ng/l, besonders bevorzugt im Bereich von 10-50 ng/l, ganz besonders bevorzugt im Bereich von 5-100 ng/l und noch stärker bevorzugt im Bereich von 1-500 ng/l verwendet.

In einer bevorzugten Ausführung des erfindungsgemäßen Verfahrens wird ein sFlt-1-*Cut-off-*Wert ≤ 37,4 ng/l als Referenzwert verwendet. In einer anderen bevorzugten Ausführung des erfindungsgemäßen Verfahrens wird ein sFlt-1-*Cut-off*-Wert ≤ 56,5 ng/l als Referenzwert verwendet. Bevorzugt wird ein sFlt-1-*Cut-off*-Wert im Bereich von 37,4-56,5 ng/l, besonders bevorzugt im Bereich von 25-100 ng/l, ganz besonders bevorzugt im Bereich von 10-250 ng/l und noch stärker bevorzugt im Bereich von 5-500 ng/l verwendet.

In einer besonders bevorzugten Ausführung des erfindungsgemäßen Verfahrens bedeutet eine Konzentration von PIGF > 17,7 ng/l eine hohe und < 17,7 ng/l eine niedrige PIGF-Konzentration. In einer alternativen, besonders bevorzugten Ausführung des erfindungsgemäßen Verfahrens bedeutet eine Konzentration von PIGF > 23,3 ng/l eine hohe, 15,6-23,3 ng/l eine mittlere und < 15,6 ng/l eine niedrige PIGF-Konzentration.

In einer besonders bevorzugten Ausführung des erfindungsgemäßen Verfahrens bedeutet eine Konzentration von sFlt-1 > 56,5 ng/l eine hohe und < 56,6 ng/l eine niedrige sFlt-1-Konzentration. In einer alternativen, besonders bevorzugten Ausführung des erfindungsgemäßen Verfahrens bedeutet eine Konzentration von sFlt-1 > 91,4 ng/l eine hohe, 37,4-91 ,4 ng/l eine mittlere und < 37,4 ng/l eine niedrige sFlt-1-Konzentration.

Das Bilden eines "Verhältnisses" zwischen PlGF und sF1t-1 kann durch Berechnung eines Quotienten von PIGF/sF1t-1 erfolgen. Alternativ kann auch ein Quotient von sFlt-1/PIGF gebildet werden. Vorzugsweise gibt ein Quotient von ≥ 0,31, ausgehend von einem Verhältnis [PIGF > 17,7 ng/l: sF1t-1 < 56,6 ng/l], ein erhöhtes Risiko für ein nachteiliges Ereignis an.

Besonders bevorzugt ist ein Quotient von ≥ 0,42, [PIGF > 15,6 ng/l: sF1t-1 < 37,4 ng/l] als Indikator für ein erhöhtes Risiko eines nachteiligen Ereignisses. Ganz besonders bevorzugt ist ein Quotient von ≥ 0,62, [PIGF > 23,3 ng/l: sFlt-1 < 37,4 ng/l] als Indikator für ein erhöhtes Risiko eines nachteiligen Ereignisses. Das Bilden eines Verhältnisses kann auch bedeuten, dass die Werte von PIGF und sFlt-1, beispielsweise durch einfaches Vergleichen, zueinander in Beziehung gesetzt werden.

In einer Ausführung umfasst das erfindungsgemäße Verfahren ein Quantifizieren von mindestens einem weiteren Biomarker. In einer bevorzugten Ausführung ist der weitere Biomarker ausgewählt aus der Gruppe, bestehend aus VEGF, sCD40L, PAPP-A (*pregnancy associated plasma protein-A*), MPO (Myeloperoxidase), Cystatin C, Myoglobin, Kreatin-Kinase, insbesondere Kreatin-Kinase MB (CK-MB, Troponin, insbesondere Troponin I, Troponin, T und/oder deren Komplexe, CRP, natriuretische Peptide, wie z.B. ANP (*atrial natriuretic peptide*), BNP (*B-type natriuretic peptide*) oder NT-proBNP. Weitere Biomarker sind auch Hämatopoietine, wie etwa EPO (Erythropoietin), GM-CSF (*granulocyte*/*macrophage colony-stimulating factor),* G-CSF *(granulocyte colony-stimulating factor),* LIF *(leukemia inhibition factor),* Oncostatin, CNTF *(ciliary neurotrophic factor),* Myoglobin, Lp-PLA₂ *(lipoprotein associated phospholipase A₂),* IMA *(Ischemia modified albumin), cysteinylated albumin,* GP-BB *(Glycogen Phosphorylase Isoenzym BB),* H-FABP *(heart-type fatty-acid-binding protein),* Cholin, PPARs *(peroxisome proliferator activator receptors),* ADMA *(asymmetric dimethylarginine),* SAA *(serum amyloid A protein),* Fibrinogen, FFAs *(unbound free fatty acids),* D-Dimer, Homocystein, PAI-1 *(plasminogen activator inhibitor 1), P-selectin, soluble E-selectin,* Hämoglobin A1c, Urodilatin, Thromboxane (beispielsweise Thromboxan A₂ und 11-dehydro-Thromboxan B₂), *mitochondrial adenylate kinase isozymes,* proMBP *(Eosinophil Major Basic Protein),* OPG (Osteoprotegerin), Leptin, Adiponectin, FSAP *(factor sevenactivating protease;* insbesondere deren sogenannte Marburg I-Mutante), IL-6 *(Interleukin-6),* MIF *(macrophage migration inhibition factor),* CALCR (Calcitonin-Rezeptor), Glycophorin (insbesondere trunkiertes Glycophorin), Wachtumshormon, Prolaktin und Interleukine, Chemokine, wie etwa Plättchenfaktor 4, *PBP (platelet basic protein),* MIP *(macrophage inflammatory protein),* Interferone, TNF (Tumornekrosefaktor), Adhäsionsmoleküle, wie etwa ICAM *(intracellular adhesion molecule)* oder VCAM *(vascular adhesion molecule),* Cytokine und weitere Wachstumsfaktoren, wie etwa FGF *(fibroblast growth factor).* Der Begriff "Biomarker" bezeichnet endogene Substanzen, z.B. Proteine, die beispielsweise das Auftreten eines pathophysiologischen Ereignisses im einem Organismus anzeigen.

In einer Ausführung besteht die Überwachung einer vaskulären Erkrankung mit atherosklerotischer Ätiologie darin, einen Patienten zu überwachen, der mit einem oder mehreren therapeutischen Mitteln behandelt wird, die das Risiko für eine vaskuläre, vorzugsweise ein kardiovaskuläre Störung reduzieren.

In einer anderen Ausführung wird das erfindungsgemäße Verfahren zur Identifizierung eines Patienten verwendet, der aus der Behandlung mit einem oder mehreren therapeutischen Mitteln, die das Risikos für eine vaskuläre, vorzugsweise ein kardiovaskuläre Störung reduzieren, voraussichtlich einen Nutzen ziehen wird. Der "Nutzen" kann darin bestehen, dass das Risiko, ein nachteiliges Ereignis, wie z.B. Tod, nicht-tödlicher Myokardinfarkt oder Schlaganfall, zu erleiden reduziert wird. Weiterhin kann der Nutzen durch eine spezifische Auswahl von Hochrisiko-Patienten durch eine individuell ausgerichteten Behandlung optimiert werden.

Die Mittel, die das Risiko für eine vaskuläre, vorzugsweise ein kardiovaskuläre Störung reduzieren, schließen solche ein, die ausgewählt sind aus der Gruppe, bestehend aus sFlt-1, entzündungshemmenden Mitteln, Antithrombotika, gegen Blutplättchen wirkende Mittel, fibrinolytische Mittel, Lipid senkende Mittel, direkte Thrombininhibitoren, und Glykoprotein IIb/IIIa-Rezeptorinhibitoren. In einer bevorzugten Ausführung ist das Mittel sFlt-1 oder von sF1t-1 abgleitet. Beispielsweise kann es sich um rekombinant hergestelltes sF1t-1, ein Fragment oder ein Derivat davon handeln.

Entzündungshemmende Mittel schließen Alclofenac, Alclometasone-Dipropionat, Algestone-Acetonide, alpha-Amylase, Amcinafal, Amcinafide, Amfenac-Natrium, Amiprilose-Hydrochlorid, Anakinra, Anirolac, Anitrazafen, Apazon, Balsalazide-Dinatrium; Bendazac, Benoxaprofen, Benzydamine-Hydrochlorid, Bromelaine, Broperamol, Budesonid, Carprofen, Cicloprofen, Cintazon, Cliprofen, Clobetasol-Propionat, Clobetason-Butyrat, Clopirac, Cloticasone-Propionate, Cormethason-Acetat, Cortodoxon, Deflazacort, Desonid, Desoximetason, Dexamethason-Dipropionat, Diclofenac-Kalium, Diclofenac-Natrium, Diflorason-Diacetat, Diflumidon-Natrium, Diflunisal, Difluprednat, Diftalon, Dimethyl-Sulfoxid, Drocinonid, Endryson, Enlimomab, Enolicam-Natrium, Epirizol, Etodolac, Etofenamate, Felbinac, Fenamol, Fenbufen, Fenclofenac, Fenclorac, Fendosal, Fenpipalon, Fentiazac, Flazalon, Fluazacort, Flufenamic-Säure, Flumizol,; Flunisolid-Acetat, Flunixin, Flunixin-Meglumine, Fluocortin-Butyl, Fluormetholon-Acetat, Fluquazon, Flurbiprofen, Fluretofen, Fluticason-Propionat, Furaprofen, Furobufen, Halcinonid, Halobetasol-Propionat, Halopredon-Acetat, Ibufenac-Ibuprofen, Ibuprofen-Aluminium, Ibuprofen-Piconol, Ilonidap, Indomethacin, Indornethacin-Natrium, Indoprofen, Indoxol, Intrazole, Isoflupredon-Acetate, Isoxepac, Isoxicam, Ketoprofen, Lofemizol-Hydrochlorid, Lomoxicam, Loteprednol-Etabonat, Meclofenamat-Natrium, Meclofenamic-Säure, Meclorison-Dibutyrat, Mefenamic-Säure, Mesalamin, Meseclazon, Methylprednisolon-Suleptanate,; Morniflumat, Nabumeton, Naproxen, Naproxen-Natrium, Naproxol, Nimazon, Olsalazin-Natrium, Orgotein, Orpanoxin, Oxaprozin, Oxyphenbutazo, Paranylin-Hydrochlorid, Pentosan-Polysulfat-Natrium, Phenbutazon-Natrium-Glycerat, Pirfenidon, Piroxicam, Piroxicam-Cinnamat, Piroxicam-Olamin, Pirprofen, Prednazat, Prifelon, Prodolic-Säure, Proquazon, Proxazol, Proxazol-Citrat, Rimexolon, Romazarit, Salcolex, Salnacedin, Salsalat, Salycilate, Sanguinarium-Chlorid, Seclazon, Sermetacin, Sudoxicam, Sulindac, Suprofen, Talmetacin, Talniflumat, Talosalat, Tebufelon, Tenidap, Tenidap-Natrium, Tenoxicam, Tesicam, Tesimid, Tetrydamin, Tiopinac, Tixocortol-Pivalat, Tolmetin, Tolmetin-Natrium, Triclonid, Triflumidat, Zidometacin, Glucocorticoide und Zomepirac-Natrium ein.

Antithrombotisch und/oder fibrinolytisch wirkende Mittel schließen Plasminogen (zu Plasmin durch Wirkung von Präkallikrein, Kininogenen, Faktor XII, Faktor XIIIa, Plasminogen-Proaktivator und Gewebeplasminogenaktivator [TPA]), Streptokinase, Urokinase, *anisoylated plasminogen-streptokinase activator complex,* Pro-Urokinase (Pro-UK); rTPA (Alteplase oder Aktivase; r = recombinant); rPro-UK; Abbokinase; Eminase; Sreptase Anagrelid-Hydrochloride, Bivalirudin, Dalteparin-Natrium, Danaparoid-Natrium, Dazoxiben-Hydrochlorid, Efegatran-Sulfat, Enoxaparin-Natrium, Ifetroban, Ifetroban-Natrium, Tinzaparin-Natrium, Retaplase, Trifenagrel, Warfarin und Dextrane ein.

Gegen Blutplättchen wirkende Mittel schließen Clopidogrel, Sulfinpyrazon, Aspirin, Dipyridamol, Clofibrat, Pyridinol-Carbamat, PGE, Glukagon, Antiserotonin-Mittel, Koffein, Theophyllin-Pentoxifyllin, Ticlopidin und Anagrelid ein.

Lipid senkende Mittel schließen Gemfibrozil, Cholystyramine, Colestipol, Nikotinsäure, Probucol-Lovastatin, Fluvastatin, Simvastatin, Atorvastatin, Pravastatin und Cirivastatin ein.

Direkte Thrombininhibitoren schließen Hirudin, Hirugen, Hirulog, Agatroban, PPACK, Thrombin-Aptamere ein.

Glykoprotein IIb/IIIa-Rezeptorinhibitoren sind sowohl Antikörper als auch Nicht-Antikörper und schließen ReoPro^{®} (abciximab), Lamifiban und Tirofiban ein, ohne darauf beschränkt zu sein.

Der Nachweis von PIGF und/oder sFlt-1 kann durch immunologische Verfahren, z.B. ELISA, erfolgen, wobei auch ein Nachweis von Bruchstücken von PIGF und/oder sFlt-1, z.B. Peptiden, sowie von PIGF und/oder sFlt-1-Isoformen und Derivaten eingeschlossen ist. Alternativ kann beispielsweise auch die mRNA von PIGF und/oder sFlt-1 nachgewiesen werden. Neben dem oben genannten ELISA können auch andere immunchemische Verfahren zur Quantifizierung von PlGF und/oder sFlt-1 erfindungsgemäß eingesetzt werden. Besonders geeignet sind heterogene oder homogene Sandwich-Immunoassays, aber auch kompetitive Immunoassays können zur Quantifizierung eingesetzt werden. Üblicherweise werden für solche Testverfahren monoklonale oder polyklonale Antikörper als spezifische Bindungspartner eingesetzt, aber anstatt der Antikörper können auch andere Substanzen (z.B. Aptamere), die spezifisch an PlGF oder sF1t-1 zu binden vermögen, eingesetzt werden. Unter dem Begriff "Antikörper" sind nicht nur komplette Antikörper zu verstehen sondern ausdrücklich auch Teile, Derivate oder Homologe von Antikörpern, wie etwa Antikörperfragmente, z.B. Fab, Fv, F(ab')₂, Fab', chimäre, humanisierte, bi- oder oligospezifische, oder *single chain*-Antikörper; des Weiteren auch Aggregate, Polymere und Konjugate von Immunglobulinen.

Die in den Immunoassays eingesetzten Antikörper oder anderen spezifischen PIGF- oder sFlt-1-Bindungspartner können an einen Träger gebunden sein, der aus porösem und/oder nicht porösem, in der Regel wasserunlöslichem Material besteht und die unterschiedlichsten Formen aufweisen kann. Der Träger kann Bestandteil einer Vorrichtung sein, wie z.B. ein Gefäß, Röhrchen, eine Mikrotitrationsplatte, Kugel, ein Mikropartikel, Stäbchen oder Streifen sowie Filter- oder Chromatographiepapier.

Die Antikörper oder anderen spezifischen PIGF- oder sFlt-1-Bindungspartner können an ein Nachweismittel *("Label")* gebunden sein, das selbst ein Signal produziert oder die Produktion eines Signals induzieren kann, wie z.B. eine fluoreszierende Substanz, eine radioaktive Substanz, ein Enzym, ein Mikropartikel (z.B. ein ungefärbter, gefärbter oder anders markierter Latexpartikel, ein Goldsolpartikel etc.) oder eine chemilumineszierende Substanz, oder das als Mittler (z.B. Biotinlabel) zu einem Nachweissystem (z.B. Avdidin-Peroxidase-Komplex) dient.

Besonders vorteilhaft im Sinne der Erfindung ist die Verwendung von Testverfahren, die die Quantifizierung von PIGF und sFlt-1 in einem Testansatz erlauben. Dies kann beispielsweise dadurch geschehen, dass man der Probe spezifische PIGF- und sFlt-1-Bindungspartner zufügt, die an unterschiedliche Nachweismittel (z.B. zwei bei unterschiedlichen Wellenlängen fluoreszierende Substanzen) gebunden sind, so dass man die nach Ablauf der immunchemischen Reaktion resultierenden Messsignale getrennt messen kann. Ein ganz besonders vorteilhafte Ausführungsform eines solchen Testverfahrens beruht auf der räumlich getrennten Messung der zur PIGF- und sFlt-1-Konzentration korrelierenden Messsignale, z.B. mittels eines immunchromatographische Testelements, wie sie im Prinzip zum Nachweis von Drogen oder Schwangerschaftshormonen verwendet werden.

Bei einer Form des erfindungsgemäßen Verfahren wird zur Quantifizierung von PlGF und sFlt-1 die Probe und, sofern nicht schon im Testelement vorzugsweise in getrockneter Form vorhanden, die markierten, d.h. die mit einem Nachweismittel assoziierten, anti-PlGFAntikörper und anti-sFlt-1-Antikörper, auf die Probenauftragszone des Testelements aufgetragen. Besonders geeignete Markierungen sind z.B. gefärbte Latexpartikel, kolloidales Gold, Enzyme, fluoreszierende Substanzen, radioaktive Substanzen oder chemilumineszierende Substanzen. Sofern PIGF und/oder sF1t-1 in der Probe enthalten sind, werden sich PlGF/Antikörper-Komplexe und/oder sFlt-1/Antikörper-Komplexe ausbilden. Diese Komplexe und evtl. noch vorhandene freie PIGF- bzw. sFlt-1-Moleküle bewegen sich z.B. mittels Kapillarkraft in Richtung auf einen Bereich (Nachweiszone) des Testelements, in dem räumlich voneinander getrennt andere anti-PIGF-Antikörper und anti-sFlt-1-Antikörper, die z.B. in Form von zwei Banden, fixiert sind oder im Laufe des Testverfahrens fixiert werden (z.B. über eine Biotin-Avidin-Brücke). Sofern in der Probe PIGF und/oder sFlt-1 vorhanden waren, werden sich in dieser Nachweiszone markierte PlGF/Antikörper-Sandwich-Komplexe und/oder markierte sFlt-1/Antikörper-Sandwich-Komplexe ausbilden. Nicht gebundene Komponenten werden vom Flüssigkeitsstrom in andere Bereiche des Testelements transportiert. Die Intensität des jeweiligen Signals in der Nachweiszone ist proportional zur PIGF- bzw. sFlt-1-Probenkonzentration. Zwar ist das oben beschriebene Sandwich-Immunoassay-Verfahren besonders bevorzugt, aber ein kompetitives Testverfahren zur Quantifizierung von PIGF und sF1t-1 auf Basis eines solchen Testelements ist ebenso möglich. Anstatt eines oder mehrerer Antikörper können - wie schon weiter oben erwähnt - auch andere Substanzen, die spezifisch an PlGF oder sFlt-1 zu binden vermögen, eingesetzt werden.

Ein weiterer Gegenstand dieser Erfindung ist daher auch ein Testelement, beispielsweise ein immunchromatisches Testelement, welches eine Probenauftragszone umfasst, bei der es sich z.B. um ein Filterpapier oder um ein anderes chromatographisches Mittel handeln kann, auf die die Probe und, sofern nicht schon im Testelement vorzugsweise in getrockneter Form vorhanden, die markierten anti-P1GF-Antikörper und anti-sFlt-1-Antikörper aufgetragen werden können, und wobei die Probenauftragszone in Kontakt zu einer Nachweiszone steht, so dass eine auf die Probenauftragszone aufgetragene Flüssigkeit die Nachweiszone, z.B. mittels Kapillarkraft, erreichen kann und die Nachweiszone räumlich getrennte Bereiche zur spezifischen Bindung von PIGF und sFLT-1 umfasst, so dass dort ggf. in der Flüssigkeit vorhandene PlGF- oder sFlt-1-Moleküle gebunden werden können. Ferner kann das Testelement auch eine mit der Nachweiszone in Kontakt stehende, vorzugsweise aus stark saugenden Material bestehende Absorptionszone (z.B. Filterpapier) umfassen, in die nicht gebundene Komponenten vom Flüssigkeitsstrom transportiert werden. In einer weiteren Ausgestaltung dieses erfindungsgemäßen Testelements weist dieses noch Mittel auf, die eine Zuordnung der Signalstärke zur PlGF- bzw. sFlt-1-Probenkonzentration, insbesondere im klinischen Entscheidungsbereich (vorzugsweise im *Cut-off-*Bereich), erlauben oder erleichtern. In einer alternativen Ausgestaltung dieses erfindungsgemäßen Testelements wird von einem kompetitiven Immunoassay anstatt vom Sandwich-Immunassay Gebrauch gemacht. In einer Ausführung der Erfindung erfolgt die Verwendung des Testelements zur Durchführung des erfindungsgemäßen Verfahrens. In einer anderen Ausführung der Erfindung erfolgt die Verwendung des Testelements zur Diagnose, Risikostratifizierung und/oder Überwachung einer vaskulären Erkrankung mit atherosklerotischer Ätiologie bei einem Patienten und/oder zur Abschätzung der Wahrscheinlichkeit für einen Patienten, eine derartige Erkrankung zu entwickeln. Anstatt eines oder mehrerer Antikörper können - wie schon weiter oben erwähnt - auch andere Substanzen, die spezifisch an PlGF oder sFlt-1 zu binden vermögen, in diesem Testelement eingesetzt werden.

Das Testelement, das ein aus einem oder mehreren Elementen zusammengesetzter Teststreifen sein kann, kann eine Probenauftragszone und jeweils eine Nachweiszone für PIGF bzw. sFlt-1 aufweisen. In einer Ausführung besteht das Testelement aus zwei parallelen Teststreifen, die jeweils aus mehreren Elementen zusammengesetzt sein können und/oder die an der Probenauftragszone oder an der Adsorptionszone miteinander in Kontakt stehen können verbunden sein können. In einer Ausführung werden zwei unabhängige Testelemente, d.h. eines für PIGF und eines für sFlt-1, verfügbar gemacht. Die Testelemente können Bestandteil eines Kits sein. In einer weiteren Ausführung wird das Testelement für das erfindungsgemäße Verfahren verwendet.

Da eine immunchemisch ermittelte Konzentration einer Substanz von den verwendeten Testverfahren und insbesondere von den verwendeten Standards und Antikörpern abhängig ist, können sich die mit zwei Tests ermittelten Konzentrationswerte einer Substanz in ein und derselben Probe durchaus unterscheiden. Sofern daher erfindungsgemäß ein Test zur Quantifizierung von PIGF oder sFlt-1 eingesetzt wird, der sich von dem in den Beispielen eingesetzten unterscheidet, empfiehlt es sich entweder, die ermittelten Konzentrationswerte unter Einbeziehung eines Umrechnungsfaktors entsprechend umzurechnen oder anhang eines geeigneten Referenzkollektivs (siehe z.B. unten "A. PATIENTEN UND METHODEN, 1. Patienten") die Referenzwerte sowie die Tertilen für den Test selbst zu bestimmen und dann diese Werte erfindungsgemäß zu verwenden. Ein Abgleich der Standards zwischen den Tests ist ebenfalls möglich.

Ein Gegenstand der Erfindung ist auch eine Referenzprobe, deren PIGF- und/oder sF1t-1-Konzentration im jeweiligen *Cut-off-*Bereich (insbesondere wie weiter unten angegeben) des erfindungsgemäßen Verfahrens liegt. Eine bevorzugte Referenzprobe weist eine PIGF-Konzentration von > 15,6 ng/l, vorzugsweise > 17,7 ng/l, besonders bevorzugt > 23,3 ng/l, und/oder eine sFlt-1-Konzentration von < 56,5 ng/l, vorzugsweise < 37,4 ng/l, auf. Bevorzugt wird auch eine Referenzprobe mit einer PIGF-Konzentration im Bereich von 15,6-23,3 ng/l, besonders bevorzugt im Bereich von 10-50 ng/l, ganz besonders bevorzugt im Bereich von 5-100 ng/l und noch stärker bevorzugt im Bereich von 1-500 ng/l. Weiterhin bevorzugt wird auch eine Referenzprobe mit einer sFlt-1-Konzentration im Bereich von 37,4-56,5 ng/l, besonders bevorzugt im Bereich von 25-100 ng/l, ganz besonders bevorzugt im Bereich von 10-250 ng/l und noch stärker bevorzugt im Bereich von 5-500 ng/l. Eine weitere bevorzugte Referenzprobe weist eine PIGF-Konzentration auf, die im Bereich des experimentell ermittelten oder z.B. vom Hersteller angegebenen PIGF-*Cut-off*-Werts ± 25%, besonders bevorzugt ± 50% und ganz besonders bevorzugt 100%, liegt. Eine weitere bevorzugte Referenzprobe weist eine sFlt-1-Konzentration auf, die im Bereich des experimentell ermittelten oder z.B. vom Hersteller angegebenen sFlt-1-*Cut-off*-Werts ± 25%, besonders bevorzugt ± 50% und ganz besonders bevorzugt ± 100%, liegt. Die Referenzprobe kann auch Mittel zur Stabilisierung von PIGF und/oder s-FlT-1 enthalten, bevorzugt Proteaseinhibitoren. In einer Ausführungsform der Erfindung wird diese erfindungsgemäße Referenzprobe in einem Verfahren zur Diagnose, Risikostratifizierung und/oder Überwachung einer vaskulären Erkrankung mit atherosklerotischer Ätiologie und/oder zur Abschätzung der Wahrscheinlichkeit, eine derartige Erkrankung zu entwickeln, verwendet.

In einer Ausführung umfasst das erfindungsgemäße Kit mindestens ein Mittel zum Quantifizieren von PIGF und mindestens ein Mittel zum Quantifizieren von sFlt-1 in einer zu untersuchen Probe, wahlweise bestehend aus separaten Packungseinheiten, wobei das Kit weiterhin mindestens eine erfindungsgemäße Referenzprobe umfasst. Die Referenzprobe kann (i) PIGF, (ii) sFLT-1 oder (iii) PlGF und sFlt-1 enthalten. Das Kit kann auch das weiter oben beschriebene Informationsmittel umfassen. Ein Kit kann auch einen oder mehrere Testelemente umfassen.

Ein diagnostisches Kit kann weitere Komponenten und/oder Hilfsstoffe umfassen. Beispielsweise kann das Kit weitere Erläuterungen zur Interpretation der Ergebnisse des Tests sowie gegebenenfalls Therapievorschläge enthalten. Auch kann das Kit ein oder mehrere Testelemente enthalten oder aus einem oder mehreren Testelementen bestehen.

### Genaue Beschreibung der Erfindung

Die vorliegende Erfindung soll im folgenden anhand von Beispielen unter Bezugnahme auf die beigefügte Figuren näher erläutert werden, ohne dass die Erfindung dadurch eingeschränkt wird. In den Figuren zeigt:
- **Figur 1**: den Zusammenhang zwischen den Plasmakonzentrationen von sFlt-1 und PLGF.
- **Figur 2**: sFlt-1-Konzentrationen bezogen auf den PIGF-Ausgangsstatus sowie PIGF-Konzentrationen bezogen auf die Ausgangskonzentration von sFlt-1.
- **Figur 3**: Ereignisraten, berechnet nach Kaplan-Meier, wobei die kumulative Inzidenz von Tod, nicht-tödlichem Myokardinfarkt, Schlaganfall und Wiederbelebung auf die Ausgangskonzentration von PlGF im Plasma bezogen wird (n=230).
Die Patienten wurden gemäß den medianen PIGF-Konzentrationen an PIGF (17,7 ng/l) in Gruppen eingeteilt.
- **Figur 4**: Ereignisraten, berechnet nach Kaplan-Meier, wobei die kumulative Inzidenz von Tod, nicht-tödlichem Myokardinfarkt, Schlaganfall und Wiederbelebung auf die Ausgangskonzentration von sFlt-1 im Plasma bezogen wird (n=230). Die Patienten wurden gemäß den medianen sFlt-1-Konzentration (56,5 ng/l) in Gruppen eingeteilt.
- **Figur 5**: die prognostische Bedeutung von PIGF für die Inzidenz von Tod, nicht tödlichem Myokardinfarkt, Schlaganfall und Wiederbelebung bezogen auf sFlt-1-Konzentrationen. Die Patienten wurden gemäß den PlGF-Konzentrationen (<15,6;15,6-23,3; >23,3 ng/l) bzw. den sFlt-1-Konzentrationen (<37,4;37,4-91,4; >91,4 ng/l) in Tertile eingeteilt (n=230).
- **Figur 6**: Ereignisraten, berechnet nach Kaplan-Meier, wobei die kumulative Inzidenz von Tod, nicht-tödlichem Myokardinfarkt, Schlaganfall und Wiederbelebung auf die Ausgangskonzentration von Flt-1- bzw. PIGF bezogen wird (n=230). Die Patienten wurden gemäß den medianen Konzentrationen an sFlt-1 und PIGF in Gruppen eingeteilt.
- **Figur 7**: Änderungen der Konzentrationen an PIGF bzw. sFlt-1 bezogen auf eine randomisierte Behandlung während der weiteren Beobachtung. Die Proben wurden zu Beginn (Basiswert), nach 30 Tagen und 12 Monaten gewonnen (n ≥ 80).

### A. PATIENTEN UND METHODEN

### 1. Patienten

Die in dieser Studie untersuchten Patienten waren solche, die bereits an der OPTIMAAL-Studie (Optimal Trial In Myocardial Infarction with Angiotensin II Antagonist Losartan) beteiligt waren und einen Myokardinfarkt erlitten hatten. Das Design und die wichtigsten Ergebnisse der OPTIMAAL-Studie wurden bereits früher beschrieben (11). Die vorliegende Studie umfasst eine Gruppe von 230 Patienten mit nachgewiesenem Myokardinfarkt und einer Dysfunktion des linken Ventrikels und/oder einem Herzversagen während der Akutphase des Myokardinfarkts. Die Patienten wurden zufallsbedingt in Gruppen eingeteilt und auf eine Dosis von Losartan (1 x 50 mg/Tag) oder Captopril (3 x 50 mg/Tag), je nach Verträglichkeit, eingestellt. Zwischen den beiden behandelten Gruppen bestanden keine wesentlichen Unterschiede im Bezug auf die Ausgangscharakteristika.

### 2. Biochemische Analyse

Den Patienten wurde morgens im nüchternen Zustand Blut abgenommen, wobei die Blutproben in pyrogenfreien Vakuumröhrchen mit EDTA gewonnen wurden. Die Röhrchen wurden sofort in Eiswasser getaucht, innerhalb von 15 Minuten zentrifugiert (1.000 g, 4°C, 15 Minuten), und das Plasma wurde in Form einer Vielzahl von Aliquots bei -80°C bis zur Analyse gelagert. Die Bestimmung der Marker wurde blind, d.h. ohne Kenntnis der Patientengeschichten und der zugewiesenen Behandlung, im Zentrallabor der Universität Frankfurt vorgenommen. PIGF, VEGF, sFlt-1 und sCD40-Ligand (sCD40L) wurden unter Anwendung der ELISA-Technik (alle Reagenzien von R&D Systems, Wiesbaden) gemessen (7, 12, 13). Hochsensitives C-reaktives Protein (hsCRP) wurde unter Verwendung des Behring BN II Nephelometers (Dade-Behring, Deerfield, Illinois) gemessen (14).

### 3. Studienendpunkte

Im Zusammenhang mit der vorliegenden Studie wurde ein Endpunkt festgelegt, der sich aus mehreren Parametern zusammensetzte. Davon umfasst waren die Gesamtmortalität, unabhängig von der Todesursache, eine Wiederbelebung nach Herzstillstand, ein wiederholter nicht-tödlichen Myokardinfarkt sowie ein Schlaganfall. Eine genaue Beschreibung des Designs und der Organisation der OPTIMAAL-Studie ist bereits früher veröffentlicht worden (11,15).

### 4. Statistische Verfahren

Es wurde ein logistisches Regressionsmodell verwendet, um das relative Risiko für vaskuläre Ereignisse zu bestimmen (16). Die Gruppeneinteilung erfolgte gemäß der medianen Konzentration jedes Biomarkers. Ein logistisches Regressionsmodell wurde verwendet, um das relative Risiko von Tod, nicht-tödlichem Myokardinfarkt, Schlaganfall und der Notwendigkeit einer Wiederbelebung zu bestimmen (16). Die Auswirkung der Ausgangscharakteristika und biochemischen Marker auf jede der untersuchten Zusammenhänge zwischen PIGF-Konzentrationen bzw. sFlt-1-Konzentrationen und vaskulären Ereignissen wurde durch schrittweise funktionierende logistische Regressionsmodelle analysiert. Alle Ergebnisse, die für kontinuierliche Variablen erhalten wurden, werden als Mittelwert ± Standardabweichung angegeben. Vergleiche zwischen den Gruppen wurden durch den t-Test (zweiseitig) analysiert. Ein Vergleich der kategorischen Variablen wurde durch den Pearson χ²-Test vorgenommen. Werte von p<0,05 wurden als statistisch signifikant betrachtet. Alle Analysen wurden unter Verwendung der Software SPSS 11,5 (SPSS Inc., Chicago, Illinois) durchgeführt.

Statistische Parameter sind: n = 230, fehlend 10; Median_{(PlGF)} = 17,7250, Median_{(sFlt-1)} = 56,5000; Perzentile = 33,33333333, 15,5700,37,4300,66,66666667,23,2700,91,4100.

Bei der Auswertung nach Kaplan-Meyer handelt es sich um ein statistisches Standardverfahren zur Berechnung von Unterschieden in der Versterbensrate oder der Rate eines ereignisfreien Überlebens.

### B. ERGEBNISSE

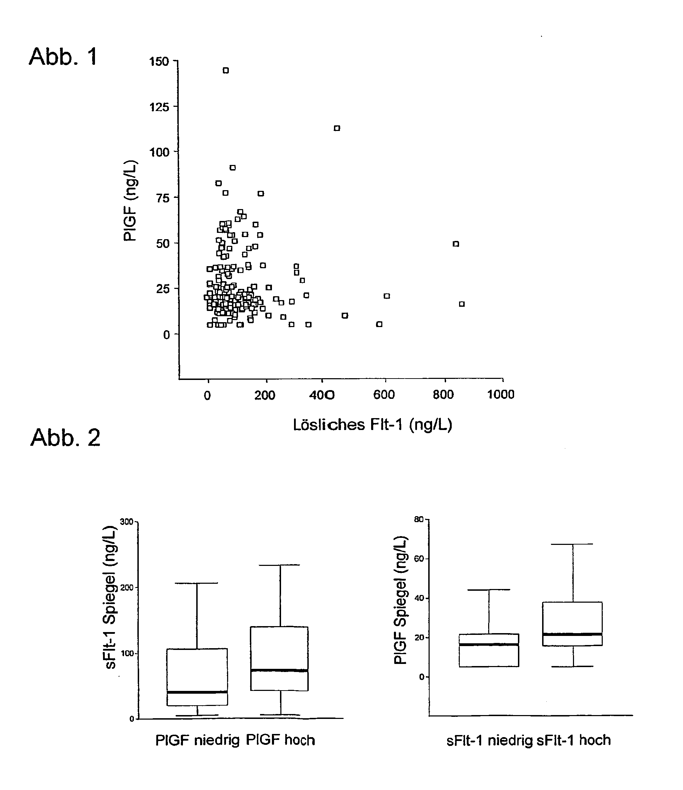

Die Ausgangskonzentrationen an sFlt-1 im Plasma zeigten einen mittleren Wert von 183,2 ± 465,6 ng/l (Bereich 5,0 bis 2503,4), und die Ausgangskonzentrationen an PIGF im Plasma betrugen 24,0 ± 20,0 ng/l (Bereich 5,0 bis 144,9). Wenn die sFlt-1-Plasmakonzentrationen mit traditionellen Biomarkern verknüpft wurden, ergab sich keine Korrelation mit hsCRP-Konzentrationen (Rangkorrelationskoeffizient nach Spearman r=-0,12; p= 0,08), wohingegen die bivariable Korrelationsanalyse eine signifikante inverse Korrelation zwischen sFlt-1 und sCD40L zeigte, obwohl die Korrelationskoeffizienten mit r=0,17 (p=0,018) niedrig waren. Darüber hinaus ergab sich keine signifikante Korrelation zwischen VEGF (r=-0,03; p=0,66) bzw. PIGF (r=0,05; p=0,44) und sFlt-1-Plasmakonzentrationen **(****Fig. 1****),** obwohl die sFlt-1-Konzentrationen bei Patienten mit erhöhtem PIGF-Konzentrationen signifikant höher waren **(****Fig. 2****).**

### Beispiel 1: Zusammenhang zwischen vaskulären Ereignissen und den Plasmankonzentrationen von PIGF und sFlt-1

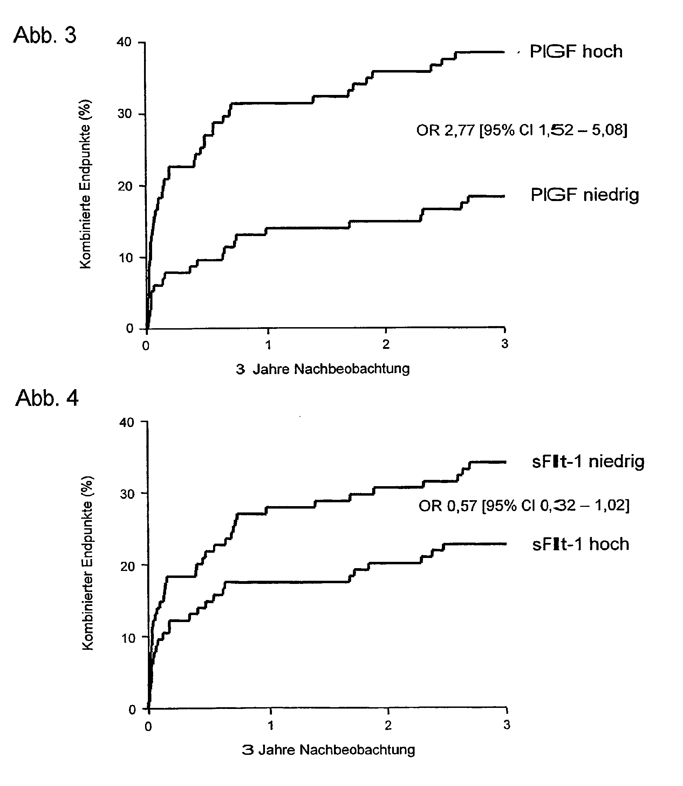

Die Patienten wurden gemäß ihren medianen Konzentrationen an Biomarkern aufgeteilt. Die Ausgangscharakteristika unterschieden sich bei Patienten mit hohen PIGF-Konzentrationen und Patienten mit niedrigen PIGF-Konzentrationen einzig im Bezug auf die sFlt-1-Konzentrationen (Tabelle 1). Bei Patienten mit erhöhten PIGF-Konzentrationen waren die Ereignisraten für die kombinierten Endpunkte Mortalität, nicht-tödlicher Myokardinfarkt, Schlaganfall und Wiederbelebung im Vergleich zu denen mit niedrigen PIGF-Konzentrationen signifikant höher (38,8% vs. 18,3%; p=0,001) **(****Fig. 3****)**. Im Bezug auf die wichtigsten vaskulären Ereignisse (Tod und nicht-tödlicher Myokardinfarkt) blieben die Unterschiede mit einer Ereignisrate von 30,4 % bei Patienten mit erhöhten PIGF-Konzentrationen im Vergleich zu 15,7 % bei Patienten mit niedrigen PIGF-Konzentrationen bestehen (Odds Ratio 2,36 [95% CI 1,24-4,48]; p=0,012).

Die Ausgangscharakteristika unterschieden sich bei Patienten mit hohen sFlt-1-Konzentrationen und Patienten mit niedrigen sFlt-1-Konzentrationen im Hinblick auf die Konzentrationen von BNP, sCD40L und PIGF sowie die Inzidenz neuer Q-Zacken im EKG und die Zeitdauer eines stationären Aufenthalts **(Tabelle 1).** Bei Patienten mit erhöhten sFlt-1-Konzentrationen waren die Ereignisraten für die kombinierten Endpunkte Mortalität, nicht-tödlicher Myokardinfarkt, Schlaganfall und Wiederbelebung tendenziell niedriger als bei Patienten mit niedrigen sFlt-1-Konzentrationen (22,6% vs. 33,9%; p=0,08) **(****Fig. 4****).** Ein nichtsignifikanter Unterschied wurde für die bedeutendsten vaskulären Ereignisse (Tod und nicht-tödlicher Myokardinfarkt) bei 19,1% der Patienten mit erhöhten sFlt-1-Konzentrationen im Vergleich zu 27,0% bei Patienten mit niedrigen sFlt-1-Konzentrationen beobachtet (Odds Ratio 0,64 [95% CI 0,34-1,19]; p=0,21).

**Tabelle 1: Basischarakteristika im Bezug auf die Plasmakonzentrationen von PIGF und sFlt-1**

| | **PLGF niedrig** | **PLGF hoch** | **sFlt-1 niedrig** | **sFlt-1 hoch** |
|---|---|---|---|---|
| N | 115 | 115 | 115 | 115 |
| Männlich | 65,2 % | 75,7 % | 66,1 % | 74,8 % |
| Alter (Jahre) | 66,6 ±10,3 | 69,0 ±10,4 | 68,7 ±10,1 | 66,9 ±10,6 |
| Neu auftretende Q-Zacken | 73,6 % | 76,6 % | 67,3 % | 83,2 % |
| Vorderwandinfarkt | 60,0 % | 61,7 % | 58,3 % | 63,5 % |
| Klassifizierung nach Killip | I: 20,0%; II | I**:** 19,1%; II | I: 15,7%; II | I: 23,5%; II |
| | 65,2 %; III | 65,2 %; III | 73,0 %; III | 57,4 %; III |
| | 13,9%; | 11,3%; | 9,6%; | 15,7%; |
| | IV 0,9% | IV 4,3% | IV 1,7% | IV 3,5% |
| Stationärer Aufenthalt | 12,1 ± 20,1 | 14,2 ± 26,4 | 17,2 ± 28,0 | 9,1 ± 17,0 * |
| (Tage) | | | | |
| Krankengeschichte | | | | |
| Angina | 19,1 % | 25,2% | 27,0 % | 17,4 % |
| Myokardinfarkt | 13,9 % | 9,6 % | 13,0 % | 10,4 % |
| PTCA | 3,5 % | 0 | 1,7 % | 1,7 % |
| CABG | 1,7 % | 0,9 % | 1,7 % | 0,9 % |
| Risikofaktoren | | | | |
| Diabetes | 11,3 % | 11,3 % | 11,3 % | 11,3 % |
| Bluthochdruck | 32,2 % | 31,3 % | 29,6 % | 33,9 % |
| Aktiver Raucher | 35,7 % | 43,5 % | 39,1 % | 40,0 % |
| Medikation | | | | |
| Aspirin | 94,8 % | 97,4 % | 95,7 % | 96,5 % |
| Statine | 61,7 % | 64,3 % | 65,2 % | 60,9 % |
| Schleifendiuretika | 74,8 % | 72,2 % | 80,9 % | 66,1 % |
| Betablocker | 76,5 % | 74,8 % | 77,4 % | 73,9 % |
| BNP (pg/ml) | 125,2 ± 93,6 | 152,3 ±126,4 | 115,5 ± 79,8 | 162,0 ± 132,9* |
| CRP (µg/ml) | 66,7 ± 66,5 | 74,0 ± 64,1 | 75,2 ± 69,7 | 65,5 ± 60,5 |
| sCD40L (pg/ml) | 4228 ± 3943 | 3915 ± 4376 | 4906 ± 4340 | 3237 ± 3809* |
| sFit-1 (pg/ml) | 108,8 ± 268 | 257,7 ± 593,5* | n.a. | n.a. |
| PIGF (pg/ml) | n.a. | n.a. | 18,5 ± 15,1 | 29,5 ± 22,7* |

### Beispiel 2: Interaktion zwischen PlGF und sFlt-1

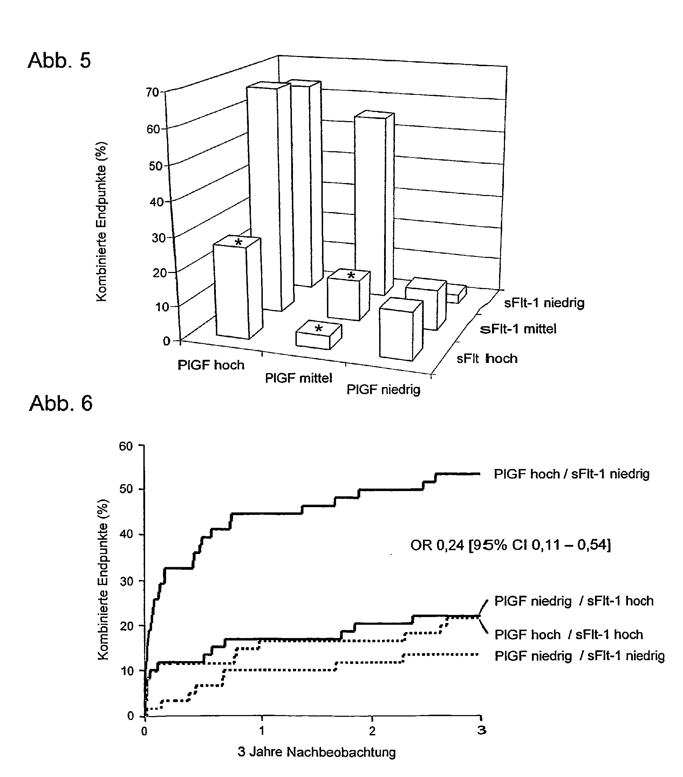

Patienten mit erhöhten PlGF-Konzentrationen wiesen auch höhere Konzentrationen an sFlt-1 auf **(****Fig. 2****).** In einem erheblichen Bereich überlappten sich die sFlt-1-Konzentrationen der beiden Gruppen jedoch, was darauf hindeutet, dass überraschenderweise der kompensatorische Anstieg der sFlt-1-Konzentrationen bei Patienten mit erhöhten PlGF-Konzentrationen uneinheitlich und nicht bei allen Patienten zu beobachten ist. Patienten mit PlGF-Konzentrationen in den oberen beiden Tertilen, die jedoch keinen Anstieg der sFlt-1-Konzentrationen (unterstes Tertil) aufwiesen, zeigten im Vergleich zu Patienten, die sFlt-Konzentrationen im obersten Tertil, aber ähnlich erhöhte PlGF-Konzentrationen aufwiesen, nachteilige Folgeerscheinungen **(****Fig. 5****).** Waren die PlGF-Konzentrationen nur leicht erhöht (zweites Tertil), schien selbst eine moderate Erhöhung der sFlt-1-Konzentrationen die Patienten vor nachteiligen Folgeerscheinungen zu schützen. Im Gegensatz dazu zeigten bei Patienten mit stark erhöhten Konzentrationen an PlGF (drittes Tertil) nur solche Patienten mit sFlt-1-Konzentrationen in dem obersten Tertil eine signifikant geringere Ereignisrate. Wenn die Patienten gemäß ihren PlGF- bzw. sFlt-1-Konzentrationen in zwei Gruppen aufgeteilt wurden, unterschied sich die Prognose der Patienten mit hohen sFlt-1-Konzentrationen nicht signifikant von den Patienten mit entweder hohen oder niedrigen PlGF-Konzentrationen **(****Fig. 6****).** Dementsprechend ist das Verhältnis von PlGF und sFlt-1 ein leistungsfähiger unabhängiger Parameter zur Vorhersage vaskulärer Ereignisse (Odds Ratio 4,00 [95% CI 2,14-7,23]; p<0,001), der der alleinigen Bestimmung eines der Parameter signifikant überlegen ist. Die Ereignisraten bei Patienten mit niedrigen PlGF-Konzentrationen betrugen 14,0% und waren von den sFlt-1-Konzentrationen (p=0,95) unabhängig. Im Gegensatz dazu betrugen die Ereignisraten bei Patienten mit hohen PlGF-Konzentrationen 55,8%, wenn die sFlt-1-Konzentrationen niedrig waren, jedoch 24,3%, wenn die sFlt-1-Konzentrationen erhöht waren (p=0,002).

Zusammenfassend lässt sich **Fig. 6** Folgendes entnehmen:
(a) Ein Verhältnis von [PlGF = hoch : sFlt-1 = niedrig] weist auf ein hohes Risiko für den Patienten auf ein für ihn nachteiliges Ereignis wie Tod, nicht-tödlicher Myokardinfarkt und Schlaganfall hin.
(b) Ist der PlGF-Wert dagegen niedrig, ist das Risiko für ein nachteiliges Ereignis deutlich verringert, und zwar unabhängig davon, ob der sFlt-1-Wert hoch oder niedrig ist.
(c) Bei einem Verhältnis von [PlGF = niedrig : sFlt-1 = niedrig] ist das Risiko für ein nachteiliges Ereignis besonders gering.
(d) Ist der sFlt-1-Wert hoch, ist das Risiko für ein nachteiliges Ereignis deutlich verringert, und zwar unabhängig davon, ob der PlGF-Wert hoch oder niedrig ist.

### Beispiel 3: Multivariable Regressionsanalyse

Um die potentielle prognostische Unabhängigkeit einzelner Biomarker weiter zu untersuchen, wurde eine schrittweise multivariable logistische Regressionsanalyse vorgenommen, die PlGF und sFlt-1 sowie weitere biochemische Marker, wie etwa BNP, einem Marker der neurohumoralen Aktivierung, hsCRP, einem klassischen Akutphaseprotein, und sCD40L, einem Marker der thrombo-inflammatorischen Aktivierung umfasste. Es wurden auch Basisscharakteristika berücksichtigt, die eine signifikante prognostische Bedeutung in einem univariablen Modell zeigten. Für die kombinierten Endpunkte nach einer 4-jährigen Beobachtungszeit erwiesen sich nur zwei etablierte Risikofaktoren, nämlich fortgeschrittenes Alter und Diabetes, als unabhängige prognostische Parameter, nachdem die biochemischen Marker in das Modell aufgenommen worden waren **(Tabelle 2).** Die Marker BNP (p=0,043), sCD40L (p=0,007), PlGF (p=0,001) und sFlt-1 (p=0,006) blieben bedeutende und unabhängige prognostische Parameter für den weiteren Krankheitsverlauf, während hsCRP etwas an Bedeutung verlor, nachdem PlGF in das Modell eingeführt worden war (p=0,77 nach Einführung von PlGF).

**Tabelle 2: Multivariates logistisches Regressionsmodel für Myokard infarkt mit tödlichem und nicht-tödlichem Ausgang im Verlauf eines 4-jährigen Nachbeobachtungszeitraums**

| **Variable** | **Odds-Ratio** | **95% Vertrauensintervall** | **p-Wert** |
|---|---|---|---|
| Alter > 75 Jahre | 2,49 | 1,13-5,47 | **0,023** |
| Diabetes mellitus | 3,06 | 1,13 - 8,29 | **0,028** |
| Hypercholesterinämie | 0,77 | 0,29 - 2,00 | 0,59 |
| BNP> 113 ng/l | 2,09 | 1,03-4,25 | **0,04** |
| C-reactives Protein > 50,0 mg/l | 1,11 | 0,55-2,25 | 0,77 |
| sCD40L > 3,5 µg/l | 2,70 | 1,31-5,58 | **0,007** |
| PlGF > 17,7 ng/l | 5,07 | 2,35-10,02 | **0,001** |
| sFlt-1 > 56,5 ng/l | 0,35 | 0,16 - 0,73 | **0,006** |
| PtGF · sFlt-1 | 3,11 | 2,03-3,88 | **0,001** |

### Beispiel 4: Änderungen der Biomarker während des Beobachtungszeitraums

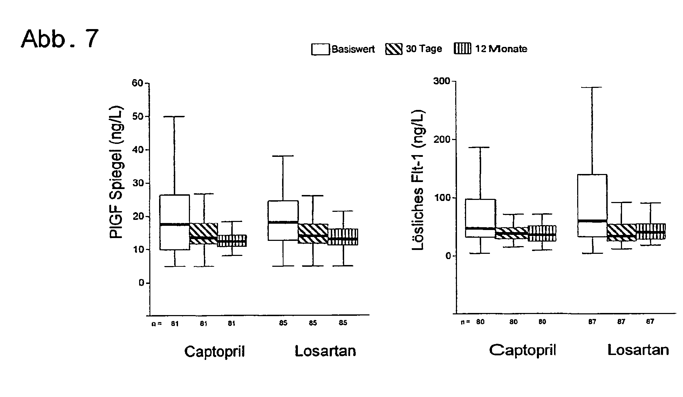

In Übereinstimmung mit den Studienergebnissen, die sich aus der Gesamtgruppe der Patienten ableiteten, ergab sich hinsichtlich des klinischen Verlaufs kein Unterschied zwischen den beiden Behandlungsgruppen (Captopril oder Losartan). Darüber hinaus wurde weder bei Patienten mit hohen noch bei Patienten mit niedrigen PlGF-Konzentrationen ein Rückgang der Ereignisse beobachtet (PlGF niedrig: 19% Ereignisrate in der Captopril-Gruppe vs. 17,5% in der Losartan-Gruppe; p=1,00; PlGF hoch: 41,1% vs. 35,6%; p=0,57). Ähnliche Ergebnisse wurden für die sFlt-1-Konzentrationen erhalten: sFlt-1 hoch: 22,2% in der Captopril-Gruppe vs. 23,9% in der Losartan-Gruppe (p=1,00); sFlt-1 niedrig: 36,7% in der Captopril-Gruppe vs. 30,9% in der Losartan-Gruppe (p=0,56). Weiterhin zeigte sich, dass bei Patienten mit verfügbaren Serienproben (Tag 0, 30 Tage und 1 Jahr; n ≥ 80 für jede Gruppe und jeden Zeitpunkt) sowohl die PlGF- als auch die sFlt-1-Konzentrationen während des Beobachtungszeitraums kontinuierlich abnahmen, wobei keine Unterschiede zwischen den Behandlungsgruppen auftraten **(****Fig. 7****).**

Die Ergebnisse der vorliegenden Studie zeigen, dass erhöhte Blutspiegel von PlGF mit vaskulären Ereignissen für Patienten nach einem Myokardinfarkt verbunden sind. In Übereinstimmung mit einer neuen Studie an Patienten mit akuten koronaren Herzerkrankungen (7) war die prognostische Bedeutung der Konzentrationen an PlGF im Plasma von anderen Biomarkern, die distinkte pathophysiologische Vorgänge repräsentieren, unabhängig. Erhöhte PlGF-Konzentrationen lieferten eine prognostische Wertigkeit, die mehr Aussagekraft hatte als Informationen, die sich von hsCRP-Plasmakonzentrationen ableiteten. Durch multivariante Regressionsanalyse wurden verschiedene andere biochemische Marker, einschließlich B-Typ natriuretisches Peptid, einem Marker einer neurohumoralen Aktivierung, sCD40L, einem Marker einer thrombo-inflammatorischen Aktivierung, und PlGF, einem Marker einer Gefäßentzündung, als unabhängige prognostische Parameter für den weiteren Verlauf der Erkrankung während der nachfolgenden 4 Jahre identifiziert. Der neue und wichtigste Befund der vorliegenden Studie ist jedoch, dass die prognostische Bedeutung von PlGF durch sFlt-1 moduliert wird. Diese Befunde belegen, dass das Gleichgewicht zwischen PlGF und seinem löslichen Rezeptor sFlt-1 als einzigem bekannten endogenen Regulator eine wesentliche Determinante hinsichtlich des weiteren Krankheitsverlauf bei Patienten mit akutem Myokardinfarkt ist.

Sowohl die Ursache der erhöhten Konzentrationen an sFlt-1 als auch die Signale, welche die Flt-1-Expression in Patienten, die einen akuten Myokardinfarkt erlitten hatten, hoch regulieren, sind derzeit nicht bekannt. Hypoxie ist ein potenter Stimulus für die Aufregulation der Flt-1-Expression (6, 19). Es ist möglich, dass ein großer Anteil von sFlt-1 durch so genanntes "*Shedding*" von den Entzündungszellen freigesetzt wird (3, 9, 20). Unabhängig von den Mechanismen, die an dem Anstieg der Konzentrationen von sFlt-1 im Plasma beteiligt sind, unterstreichen die Ergebnisse der vorliegenden Studie die Schlüsselrolle, die dem Gleichgewicht zwischen pro- und anti-inflammatorischen Mediatoren für die Risikostratifizierung im Rahmen einer akuten koronaren Herzerkrankung zukommt (21).

Vor allem aber lassen diese Studien hoffen, dass netto anti-inflammatorische Strategien entwickelt werden können, um der Progression einer bestehenden Atherosklerose entgegen zu wirken. Die Infusion von sFlt-1 mit dem Ziel, die Konzentrationen an zirkulierendem aktivem PlGF bei Patienten mit instabiler oder rasch fortschreitender koronarer Herzerkrankung zu reduzieren, könnte bei solchen Patienten, die erhöhte PlGF-Konzentrationen und niedrige Konzentrationen von dessen Inhibitor sFlt-1 aufweisen, besonders wirksam sein.

Die Ergebnisse der vorliegenden Studie zeigen, dass erhöhte Plasmakonzentrationen an PlGF, einem Marker für eine Gefäßentzündung, bei Patienten nach Myokardinfarkt mit einem erhöhten Risiko für nachfolgende vaskuläre Ereignisse verbunden sind. Die prognostische Aussagefähigkeit hängt jedoch von der Konzentration an sFlt-1 ab, was die Hypothese stützt, dass sFlt-1 die Aktivität von PlGF durch Bindung und Inaktivierung reguliert. Diese Befunde könnten die Grundlage für einen neuen anti-inflammatorischen therapeutischen Ansatz liefern, bei dem sFlt-1 verwendet wird, um zirkulierenden PlGF bei Patienten, die ein erhöhtes Risiko für ein nachteiliges vaskuläres Ereignis aufweisen, zu reduzieren.

### Literaturverzeichnis

1. Braunwald E. Unstable angina: an etiologic approach to management. Circulation. 1998;98:2219-22.
2. Libby P, Ridker PM, Maseri A. Inflammation and atherosclerosis. Circulation. 2002;105:1135-43.
3. Luttun A, Tjwa M, Moons L, Wu Y, Angelillo-Scherrer A, Liao F, Nagy JA, Hooper A, Priller J, De Klerck B, Compemolle V, Daci E, Bohlen P, Dewerchin M, Herbert JM, Fava R, Matthys P, Carmeliet G, Collen D, Dvorak HF, Hicklin DJ, Carmeliet P. Revascularization of ischemic tissues by P1GF treatment, and inhibition of tumor angiogenesis, arthritis and atherosclerosis by anti-Fltl . Nat Med. 2002;8:831-40.
4. Maglione D, Guerriero V, Viglietto G, Delli-Bovi P, Persico MG. Isolation of a human placenta cDNA coding for a protein related to the vascular permeability factor. Proc Natl Acad Sci USA. 1991;88:9267-71.
5. Autiero M, Luttun A, Tjwa M, Carmeliet P. Placental growth factor and its receptor, vascular endothelial growth factor receptor-1: novel targets for stimulation of ischemic tissue revascularization and inhibition of angiogenic and inflammatory disorders. J Thromb Haemost. 2003;1:1356-70.
6. Luttun A, Tjwa M, Carmeliet P. Placental Growth Factor (PlGF) and Its Receptor Flt-1 (VEGFR-1): Novel Therapeutic Targets for Angiogenic Disorders. Ann N Y Acad Sci. 2002;979:80-93.
7. Heeschen C, Dimmeler S, Fichtlscherer S, Hamm CW, Berger J, Simoons ML, Zeiher AM. Prognostic value of placental growth factor in patients with acute chest pain. Jama. 2004;291:435-41.
8. Khaliq A, Dunk C, Jiang J, Shams M, Li XF, Acevedo C, Weich H, Whittle M, Ahmed A. Hypoxia down-regulates placenta growth factor, whereas fetal growth restriction up-regulates placenta growth factor expression: molecular evidence for "placental hyperoxia" in intrauterine growth restriction. Lab Invest. 1999;79:151-70.
9. Rafii S, Avecilla S, Shmelkov S, Shido K, Tejada R, Moore MA, Heissig B, Hattori K. Angiogenic factors reconstitute hematopoiesis by recruiting stern cells from bone marrow microenvironment. Ann N Y Acad Sci. 2003;996:49-60.
10. Chung NA, Makin AJ, Lip GY. Measurement of the soluble angiopoietin receptor tie-2 in patients with coronary artery disease: development and application of an immunoassay. Eur J Clin Invest. 2003;33:529-35.
11. Dickstein K, Kjekshus J. Effects of losartan and captopril on mortality and morbidity in high-risk patients after acute myocardial infarction: the OPTIMAAL randomised trial. Optimal Trial in Myocardial Infarction with Angiotensin II Antagonist Losartan. Lancet. 2002;360:752-760.
12. Heeschen C, Dimmeler S, Hamm CW, van den Brand MJ, Boersma E, Zeiher AM, Simoons ML. Soluble CD40 ligand in acute coronary syndromes. N Engl J Med. 2003;348:1104-11.
13. Heeschen C, Dimmeler S, Hamm CW, Boersma E, Zeiher AM, Simoons ML. Prognostic significance of angiogenic growth factor serum levels in patients with acute coronary syndromes. Circulation. 2003;107:524-530.
14. Heeschen C, Hamm CW, Bruemmer J, Simoons ML. Predictive value of C-reactive protein and troponin T in patients with unstable angina: a comparative analysis. CAPTURE Investigators. Chimeric c7E3 AntiPlatelet Therapy in Unstable angina REfractory to standard treatment trial. J Am Coll Cardiol. 2000;35:1535-42.
15. Dickstein K, Kjekshus J. Comparison of the effects of losartan and captopril on mortality in patients after acute myocardial infarction: the OPTIMAAL trial design. Optimal Therapy in Myocardial Infarction with the Angiotensin II Antagonist Losartan. Am J Cardiol. 1999;83:477-81.
16. Cox DR Regression models and life-tables. JR Stat Soc [B]. 1972;34:187-220.
17. Maynard SE, Jiang-Yong Min J-Y, Merchan J, Lim KH, Li J, Mondal S, Libermann TA, Morgan JP, Sellke FW, Stillman IE, Epstein FH, Sukhatme VP, Karumanchi SA. Excess placental soluble fms-like tyrosine kinase 1 (sFltl)may contribute to endothelial dysfunction, hypertension, and proteinuria in preeclampsia. J. Clin. lnvest. 2003;111:649-658.
18. Levine RJ, Maynard SE, Qian C, Lim KH, England LJ, Lu KF, Schisterman EF, Thadhani R, Sachs BP, Epstein FH, Sibai BM, Sukhatme VP, Karumanchi SA. Circulating Angiogenic Factors and the Risk of Peeclampsioa. N Engl J Med. 2004;350:672-683.
19. Takeda N, Maemura K, Imai Y, Harada T, Kawanami D, Nojiri T, Manabe I, Nagai R. Endothelial PAS domain protein 1 gene promotes angiogenesis through the transactivation of both vascular endothelial growth factor and its receptor, Flt-1. Circ Res. 2004;95:146-53.
20. Selvaraj SK, Giri RK, Perelman N, Johnson C, Malik P, Kalra VK. Mechanism of monocyte activation and expression of proinflammatory cytochemokines by placenta growth factor. Blood. 2003;102:1515-24.
21. Heeschen C, Dimmeler S, Hamm CW, Fichtlscherer S, Boersma E, Simoons ML, Zeiher AM. Serum level of the antiinflammatory cytokine interleukin-10 is an important prognostic determinant in patients with acute coronary syndromes. Circulation. 2003;107:2109-14.
22. Ridker PM. Clinical Application of C-Reactive Protein for Cardiovascular Disease Detection and Prevention. Circulation 2003; 107:363-369.
23. Koenig W, Lowel H, Baumert J, Meisinger C. C-reactive protein modulates risk prediction based on the Framingham Score: implications for future risk assessment: results from a large cohort study in southern Germany. Circulation 2004; 109:1349-53.
24. Danesh J, Wheeler JG, Hirschfield GM, et al. C-reactive protein and other circulating markers of inflammation in the prediction of coronary heart disease. N Engl J Med 2004; 350:1387-97.
25. Morrow DA, Rifai N, Antman EM, et al. C-reactive protein is a potent predictor of mortality independently of and in combination with troponin T in acute coronary syndrome: a TIMI 11A substudy. Thrombolysis in Myocardial Infarction. J Am Coll Cardiol 1998; 31:1460-5.
26. Lindahl B, Toss H, Siegbahn A, Venge P, Wallentin L. Markers of myocardial damage and inflammation in relation to long-term mortality in unstable coronary artery disease. FRISC Study Group. Fragmin during Instability in Coronary Artery Disease. N Engl J Med 2000; 343:1139-47.
27. James SK, Lindahl B, Siegbahn A, et al. N-terminal pro-brain natriuretic peptide and other risk markers for the separate prediction ofmortality and subsequent myocardial infarction in patients with unstable coronary artery disease: a Global Utilization of Strategies To Open occluded arteries (GUSTO)-IV substudy. Circulation 2003; 108:275-81.

## Patentansprüche

1. Verwendung eines *in vitro*-Verfahrens, das folgende Schritte umfasst:
(a) Bereitstellen einer zu untersuchenden Probe eines Patienten;
(b) Quantifizieren von PlGF in der Probe;
(c) Quantifizieren von sFlt-1 in der Probe;
zur Diagnose, Risikostratifizierung und/oder Überwachung einer vaskulären Erkrankung mit atherosklerotischer Ätiologie und/oder zur Abschätzung der Wahrscheinlichkeit, eine derartige Erkrankung zu entwickeln.

2. Verwendung nach Anspruch 1, wobei das Verfahren weiterhin folgenden Schritt umfasst:
(d) Vergleich der in (b) und (c) ermittelten Werte von PlGF und sFlt-1 mit jeweils einem Referenzwert und/oder jeweils einem in einer Referenzprobe ermittelten
Wert;
und/oder folgende Schritte umfasst:
(d') Bilden eines Verhältnisses zwischen dem in (b) ermittelten Wert von PlGF und
dem in (c) ermittelten Wert von sFlt-1;
(e') Vergleich des in (d') ermittelten Werts mit einem Referenzwert und/oder einem in einer Referenzprobe ermittelten Wert.

3. Verwendung nach Anspruch 1 oder 2, wobei die vaskuläre Erkrankung eine koronare Herzerkrankung, cerebrovaskuläre Erkrankung und/oder eine periphere arterielle Verschlusskrankheit ist.

4. Verwendung nach Anspruch 3, wobei die koronare Herzerkrankung ein akutes Koronarsyndrom ist, vorzugsweise instabile Angina pectoris oder akuter Myokardinfarkt.

5. Verwendung nach einem der vorangehenden Ansprüche, wobei die zu untersuchende Probe peripheres Blut oder eine Fraktion davon ist, vorzugsweise Blutplasma oder Blutserum.

6. Verwendung nach einem der vorangehenden Ansprüche, wobei die Risikostratifizierung ein Bestimmen einer Wahrscheinlichkeit für ein nachteiliges Ereignis umfasst, bestehend aus Tod, nicht-tödlichem Myokardinfarkt und/oder Schlaganfall.

7. Verwendung nach Anspruch 6, wobei ein PlGF-Wert oberhalb eines PlGF-Referenzwerts von vorzugsweise > 15,6 ng/l, besonders bevorzugt > 17,7 ng/l, ganz besonders bevorzugt > 23,3 ng/l, und ein sFlt-1-Wert unterhalb eines sFlt-1-Referenzwerts von vorzugsweise < 56,5 ng/l, besonders bevorzugt < 37,4 ng/l, eine erhöhte Wahrscheinlichkeit für ein nachteiliges Ereignis anzeigt.

8. Verwendung nach einem Anspruch 6 oder 7, wobei eine P1GF-Konzentration, welche in den oberen beiden Tertilen eines Referenzkollektivs liegt, und eine sFlt-1-Konzentration, welche im untersten Tertil des Referenzkollektivs liegt, eine erhöhte Wahrscheinlichkeit für ein nachteiliges Ereignis anzeigt.

9. Verwendung nach einem der Ansprüche 6 bis 8, wobei ein Verhältnis von [PlGF = hoch : sFlt-1 = niedrig] eine erhöhte Wahrscheinlichkeit für ein nachteiliges Ereignis anzeigt.

10. Verwendung nach Anspruch 9, wobei eine Konzentration von PlGF > 15,6 ng/l, vorzugsweise > 17,7 ng/l, besonders bevorzugt > 23,3 ng/l, eine hohe PlGF-Konzentration bedeutet.

11. Verwendung nach Anspruch 9 oder 10, wobei eine Konzentration von sFlt-1 < 56,5 ng/l, vorzugsweise < 37,4 ng/l, eine niedrige sFlt-1-Konzentration bedeutet.

12. Verwendung nach einem der Ansprüche 9 bis 11, wobei ein Verhältnis von [PlGF : sFlt-1] ≥ 0,31, vorzugsweise ≥ 0,42, besonders bevorzugt ≥ 0,62 eine erhöhte Wahrscheinlichkeit für ein nachteiliges Ereignis anzeigt.

13. Verwendung nach einem der vorangehenden Ansprüche, wobei das Verfahren ein Quantifizieren von mindestens einem weiteren Biomarker umfasst, vorzugsweise VEGF, sCD40L, PAPP-A, MPO, Myoglobin, Kreatin-Kinase, insbesondere CK-MB, Troponin, insbesondere Troponin I, Troponin T und/oder deren Komplexe, CRP, Cystatin C, natriuretische Peptide, insbesondere ANB, BNP und/oder NT-proBNP.

14. In vitro-Verfahren zur Diagnose, Risikostratifizierung und/oder Überwachung einer vaskulären Erkrankung mit atherosklerotischer Ätiologie und/oder zur Abschätzung der Wahrscheinlichkeit, eine derartige Erkrankung zu entwickeln, wobei das Verfahren folgende Schritte umfasst:
(a) Bereitstellen einer zu untersuchenden Probe eines Patienten;
(b) Quantifizieren von PlGF in der Probe;
(c) Quantifizieren von sFlt-1 in der Probe.

15. Verfahren nach Anspruch 14, wobei das Verfahren weiterhin folgenden Schritt umfasst:
(d) Vergleich der in (b) und (c) ermittelten Werte von PlGF und sFlt-1 mit jeweils einem Referenzwert und/oder jeweils einem in einer Referenzprobe ermittelten
Wert;
und/oder folgende Schritte umfasst:
(d') Bilden eines Verhältnisses zwischen dem in (b) ermittelten Wert von PlGF und
dem in (c) ermittelten Wert von sFlt-1;
(e') Vergleich des in (d') ermittelten Werts mit einem Referenzwert und/oder einem in einer Referenzprobe ermittelten Wert.

16. Verfahren nach einem der Ansprüche 14 oder 15, wobei der Patient eine vaskuläre Erkrankung, bestehend aus koronarer Herzerkrankung, cerebrovaskulärer Erkrankung und/oder peripherer arterieller Verschlusskrankheit, aufweist oder im Verdacht steht, die genannte Erkrankung aufzuweisen oder zu entwickeln.

17. Verfahren nach Anspruch 16, wobei die koronare Herzerkrankung ein akutes Koronarsyndrom ist, vorzugsweise instabile Angina pectoris oder akuter Myokardinfarkt.

18. Verfahren nach einem der Ansprüche 14 bis 17, wobei der Patient mit einem oder mehreren therapeutischen Mitteln behandelt wird, bestehend aus sFlt-1, entzündungshemmenden Mitteln, Antithrombotika, gegen Blutplättchen wirkenden Mitteln, fibrinolytischen Mitteln, Lipid senkenden Mitteln, direkten Thrombininhibitoren und/oder Glykoprotein IIb/IIIa-Rezeptorinhibitoren.

19. Verfahren nach einem der Ansprüche 14 bis 18, wobei die zu untersuchende Probe peripheres Blut oder eine Fraktion davon ist, vorzugsweise Blutplasma oder Blutserum.

20. Verfahren nach einem der Ansprüche 14 bis 19, wobei das Verfahren weiterhin eine Risikostratifizierung mittels Bestimmen einer Wahrscheinlichkeit für ein nachteiliges Ereignis umfasst, bestehend aus Tod, nicht-tödlichem Myokardinfarkt und/oder Schlaganfall.

21. Verfahren nach Anspruch 20, wobei ein PlGF-Wert oberhalb eines PlGF-Referenzwerts von vorzugsweise > 15,6 ng/l, besonders bevorzugt > 17,7 ng/l, ganz besonders bevorzugt > 23,3 ng/l, und ein sFlt-1-Wert unterhalb eines sFlt-1-Referenzwerts von vorzugsweise < 56,5 ng/l, besonders bevorzugt < 37,4 ng/l, eine erhöhte Wahrscheinlichkeit für ein nachteiliges Ereignis anzeigt.

22. Verfahren nach Anspruch 20 oder 21, wobei eine PlGF-Konzentration, welche in den oberen beiden Tertilen eines Referenzkollektivs liegt, und eine sFlt-1-Konzentration, welche im untersten Tertil des Referenzkollektivs liegt, eine erhöhte Wahrscheinlichkeit für ein nachteiliges Ereignis anzeigt.

23. Verfahren nach einem der Ansprüche 20 bis 22, wobei ein Verhältnis von [PlGF = hoch : sFlt-1 = niedrig] eine erhöhte Wahrscheinlichkeit für ein nachteiliges Ereignis anzeigt.

24. Verfahren nach Anspruch 23, wobei eine Konzentration von PlGF > 15,6 ng/l, vorzugsweise > 17,7 ng/l, besonders bevorzugt > 23,3 ng/l, eine hohe PlGF-Konzentration bedeutet.

25. Verfahren nach Anspruch 23 oder 24, wobei eine Konzentration von sFlt-1 < 56,5 ng/l, vorzugsweise < 37,4 ng/l eine niedrige sFlt-1-Konzentration bedeutet.

26. Verfahren nach einem der Ansprüche 23 bis 25, wobei ein Verhältnis von [PlGF : sFlt-1] ≥ 0,31, vorzugsweise ≥ 0,42, besonders bevorzugt ≥ 0,62 eine erhöhte Wahrscheinlichkeit für ein nachteiliges Ereignis anzeigt.

27. Verfahren nach einem der Ansprüche 14 bis 26, wobei das Verfahren ein Quantifizieren von mindestens einem weiteren Biomarker umfasst, vorzugsweise VEGF, sCD40L, PAPP-A, MPO, Myoglobin, Kreatin-Kinase, insbesondere CK-MB, Troponin, insbesondere Troponin I, Troponin T und/oder deren Komplexe, CRP, Cystatin C, natriuretische Peptide, insbesondere ANB, BNP und/oder NT-proBNP.

28. Verwendung eines Verfahrens nach einem der Ansprüche 14 bis 27 zur Identifizierung eines Patienten, der voraussichtlich von der Behandlung mit einem oder mehreren therapeutischen Mitteln, bestehend aus sFlt-1, entzündungshemmenden Mitteln, Antithrombotika, gegen Blutplättchen wirkenden Mitteln, fibrinolytischen Mitteln, Lipid senkenden Mitteln, direkten Thrombininhibitoren und/oder Glykoprotein IIb/IIIa-Rezeptorinhibitoren, Nutzen ziehen wird.

29. Diagnostisches Kit, umfassend mindestens ein Mittel zum Quantifizieren von PlGF und mindestens ein Mittel zum Quantifizieren von sFlt-1 in einer zu untersuchen Probe, wahlweise bestehend aus separaten Packungseinheiten, wobei das Kit weiterhin mindestens eine Referenzprobe mit einer Konzentration von PlGF > 15,6 ng/l, vorzugsweise > 17 7 ng/l, besonders bevorzugt > 23,3 ng/l, und einer Konzentration von sFlt-1 < 56,5 ng/l vorzugsweise < 37,4 ng/l, umfasst, und wahlweise weiterhin ein Informationsmittel umfasst, wonach (i) eine PlGF-Konzentration, welche in den oberen beiden Tertilen eines Referenzkollektivs liegt, und eine sFlt-1-Konzentration, welche im untersten Tertil des Referenzkollektivs liegt, und/oder (ii) ein PlGF-Wert oberhalb des PlGF-Referenzwerts und ein sFlt-1-Wert unterhalb des sFlt-1-Referenzwerts eine erhöhte Wahrscheinlichkeit für ein nachteiliges Ereignis anzeigt, bestehend aus Tod, nicht-tödlicher Myokardinfarkt und/oder Schlaganfall.

30. Verwendung eines Kits nach Anspruch 29 zur Diagnose, Risikostratifizierung und/oder Überwachung einer vaskulären Erkrankung mit atherosklerotischer Ätiologie und/oder zur Abschätzung der Wahrscheinlichkeit, eine derartige Erkrankung zu entwickeln, oder zur Durchführung des Verfahrens nach einem der Ansprüche 1 bis 28.

31. Verwendung eines diagnostischen Kits, umfassend mindestens ein Mittel zum Quantifizieren von PlGF und mindestens ein Mittel zum Quantifizieren von sFlt-1 in einer zu untersuchen Probe, wahlweise bestehend aus separaten Packungseinheiten, wobei das Kit weiterhin ein Mittel zur Information umfasst, wonach (i) eine PlGF-Konzentration, welche in den oberen beiden Tertilen eines Referenzkollektivs liegt, und eine sFlt-1-Konzentration, welche im untersten Tertil des Referenzkollektivs liegt, und/oder (ii) ein PlGF-Wert oberhalb des PlGF-Referenzwerts und ein sFlt-1-Wert unterhalb des sFlt-1-Referenzwerts eine erhöhte Wahrscheinlichkeit für ein nachteiliges Ereignis anzeigt, ausgewählt aus der Gruppe, bestehend aus Tod, nicht-tödlichem Myokardinfarkt oder Schlaganfall, zur Diagnose, Risikostratifizierung und/oder Überwachung einer vaskulären Erkrankung mit atherosklerotischer Ätiologie und/oder zur Abschätzung der Wahrscheinlichkeit, eine derartige Erkrankung zu entwickeln, oder zur Durchführung des Verfahrens nach einem der Ansprüche 1 bis 28.

32. Verwendung eines Testelements, umfassend eine Probenauftragszone zur Auftragung der Probe und von markierten spezifischen PlGF-Bindungspartnern und sFlt-1-Bindungspartnem, wobei die Bindungspartner wahlweise im Testelement vorhanden sind, und wobei die Probenauftragszone in Kontakt zu mindestens einer Nachweiszone steht und die Nachweiszone räumlich getrennte Bereiche zur spezifischen Bindung von PlGF und sFLT-1 umfasst, zur Diagnose, Risikostratifizierung und/oder Überwachung einer vaskulären Erkrankung mit atherosklerotischer Ätiologie und/oder zur Abschätzung der Wahrscheinlichkeit, eine derartige Erkrankung zu entwickeln, und/oder zur Identifizierung eines Patienten, der voraussichtlich von der Behandlung mit einem oder mehreren therapeutischen Mitteln, bestehend aus sFlt-1, entzündungshemmenden Mitteln, Antithrombotika, gegen Blutplättchen wirkenden Mitteln, fibrinolytischen Mitteln, Lipid senkenden Mitteln, direkten Thrombininhibitoren und/oder Glykoprotein IIb/IIIa-Rezeptorinhibitoren, Nutzen ziehen wird.

33. Verwendung eines immunchromatischen Testelements, umfassend mindestens ein Mittel zum Quantifizieren von PlGF und mindestens ein Mittel zum Quantifizieren von sFlt-1 in einer zu untersuchen Probe, zur Diagnose, Risikostratifizierung und/oder Überwachung einer vaskulären Erkrankung mit atherosklerotischer Ätiologie und/oder zur Abschätzung der Wahrscheinlichkeit, eine derartige Erkrankung zu entwickeln, und/oder zur Identifizierung eines Patienten, der voraussichtlich von der Behandlung mit einem oder mehreren therapeutischen Mitteln, bestehend aus sFlt-1, entzündungshemmenden Mitteln, Antithrombotika, gegen Blutplättchen wirkenden Mitteln, fibrinolytische Mitteln, Lipid senkenden Mitteln, direkten Thrombininhibitoren und/oder Glykoprotein IIb/IIIa-Rezeptorinhibitoren, Nutzen ziehen wird.

## Claims

1. Use of an *in vitro* method comprising the following steps:
(a) providing a sample to be analyzed from a patient;
(b) quantifying PlGF in said sample;
(c) quantifying sFlt-1 in said sample;
for a diagnosis, risk stratification and/or monitoring of a vascular disease with atherosclerotic etiology, and/or for estimating the probability to develop such a disease.

2. Use according to claim 1, wherein said method further comprises the following step:
(d) comparing each of the values for PlGF and sFlt-1 as obtained in (b) and (c) with a reference and/or to a value determined in a reference sample;
and/or comprises the following steps:
(d') determining a ratio of the value for PlGF determined in (b) and the value for sFlt-1 determined in (c);
(e') comparing the value determined in (d') to a reference value and/or to a value
determined in a reference sample.

3. Use according to claim 1 or 2, wherein the vascular disease is a coronary heart disease, a cerebrovascular disease and/or a peripheral arterial occlusive disease.

4. Use according to claim 3, wherein the coronary heart disease is an acute coronary syndrome, preferably unstable angina pectoris or acute myocardial infarction.

5. Use according to any of the preceding claims, wherein the sample to be analyzed is peripheral blood or a fraction thereof, preferably blood plasma or blood serum.

6. Use according to any of the preceding claims, wherein the risk stratification comprises determining a probability of an adverse event, consisting of death, non-fatal myocardial infarction and/or stroke.

7. Use according to claim 6, wherein a PlGF value above a PlGF reference value preferably of > 15.6 ng/l, particularly preferred of > 17.7 ng/l, most particularly preferred of > 23.3 ng/l, and an sFlt-1 result below an sFlt-1 reference value preferably of < 56.5 ng/l, particularly preferred of < 37.4 ng/l indicates an elevated probability for an adverse event.

8. Use according to claim 6 or 7, wherein a PlGF concentration in the upper two tertiles of a reference collective, and an sFlt-1 concentration in the lower tertile of the reference collective indicates an elevated probability of an adverse event.

9. Use according to any of claims 6 to 8, wherein a ratio of [PlGF = high : sFlt-1 = low] indicates an elevated probability for an adverse event.

10. Use according to claim 9, wherein a concentration of PlGF of > 15.6 ng/l, preferably of > 17.7 ng/l, particularly preferred of > 23.3 ng/l, means a high PlGF concentration.

11. Use according to claim 9 or 10, wherein a concentration of sFlt-1 of < 56.5 ng/l, preferably of < 37.4 ng/l, means a low sFlt-1 concentration.

12. Use according to any of claims 9 to 11, wherein a ratio of [PlGF : sFlt-1] of ≥ 0.31, preferably of ≥ 0.42, particularly preferred of ≥ 0.62, indicates an elevated probability for an adverse event.

13. Use according to any of the preceding claims, the method comprising a quantification of at least one additional biomarker, preferably VEGF, sCD40L, PAPP-A, MPO, myoglobin, creatine kinase, particularly CK-MB, troponin, particulary troponin I, troponin T and/or their complexes, CRP, cystatin C, natriuretic peptides, particularly ANB, BNP and/or NT-proBNP.

14. An *in vitro* method for a diagnosis, risk stratification and/or monitoring of a vascular disease with atherosclerotic etiology, and/or for estimating the probability to develop such a disease, the method comprising the following steps:
(a) providing a sample to be analyzed from a patient;
(b) quantifying PlGF in said sample;
(c) quantifying sFlt-1 in said sample.

15. The method according to claim 14, said method further comprising the following step:
(d) comparing each of the values of PlGF and sFlt-1 determined in (b) and (c) to a reference and/or to a value determined in a reference sample;
and/or comprising the following steps:
(d') calculating a ratio of the value of PlGF determined in (b) and the value of sFlt-1 determined in (c);
(e') comparing the value determined in (d') to a reference and/or to a value determined in a reference sample.

16. The method according to claim 14 or 15, wherein the patient is suffering from a vascular disease consisting of coronary heart disease, cerebrovascular disease and/or peripheral arterial occlusive disease, or is suspected to suffer from or to develop the aforementioned diseases.

17. The method according to claim 16, wherein the coronary heart disease is an acute coronary syndrome, preferably unstable angina pectoris or acute myocardial infarction.

18. The method according to any of claims 14 to 17, wherein the patient is treated by one or more therapeutic agents, consisting of sFlt-1, anti-inflammatory agents, anti-thrombotics, anti-platelet agents, fibrinolytic agents, lipid lowering agents, direct thrombin inhibitors, and/or glykoprotein IIb/IIIa receptor inhibitors.

19. The method according to any of claims 14 to 18, wherein the sample to be analyzed is peripheral blood or a fraction thereof, preferably blood plasma or blood serum.

20. The method according to any of claims 14 to 19, the method further comprising a risk stratification by means of determining a probability for an adverse event, consisting of death, non-fatal myocardial infarctions and/or stroke.

21. The method according to claim 20, wherein a PlGF value above a PlGF reference value preferably of > 15.6 ng/l, particularly preferred of > 17.7 ng/l, most particularly preferred of > 23.3 ng/l, and an sFlt-1 value below an sFlt-1 reference value preferably of < 56.5 ng/l, particularly preferred of < 37.4 ng/l, indicates an elevated probability of an adverse event.

22. The method according to claim 20 or 21, wherein a PlGF concentration in the above two tertiles of a reference collective, and an sFlt-1 concentration in the lower tertile of the reference collective indicates an elevated probability for an adverse event.

23. The method according to any of claims 20 to 22, wherein a ratio of [PlGF = high :
sFlt-1 = low] indicates an elevated probability for an adverse event.

24. The method according to claim 23, wherein a concentration of PlGF of > 15.6 ng/l, preferably of > 17.7 ng/l, particularly preferred of > 23.3 ng/l, means a high PlGF concentration.

25. The method according to claim 23 or 24, wherein a concentration of sFlt-1 of < 56.5 ng/l, preferably of < 37.4 ng/l, means a low sFlt-1 concentration.

26. The method according to any of claims 23 to 25, wherein a ratio of [PlGF : sFlt-1] of ≥ 0.31, preferably of ≥ 0.42, particularly preferred of ≥ 0.62, indicates an elevated probability for an adverse event.

27. The method according to any of claims 14 to 26, the method comprising a quantification of at least one additional biomarker, preferably VEGF, sCD40L, PAPP-A, MPO, myoglobin, creatine kinase, particularly CK-MB, troponin, particularly troponin I, troponin T and/or their complexes, CRP, cystatin C, natriuretic peptides, particularly ANB, BNP and/or NT-proBNP.

28. Use of a method according to any of claims 14 to 27 for the identification of a patient who is supposed to benefit from a treatment by one or more therapeutic agents, consisting of sFlt-1, anti-inflammatory agents, anti-thrombotics, anti-platelet agents, fibrinolytic agents, lipid lowering agents, direct thrombin inhibitors, and/or glycoprotein IIb/IIIa receptor inhibitors.

29. A diagnostic kit, comprising at least one means for quantifying PlGF and at least one means for quantifying sFlt-1 in a sample to be analyzed, optionally consisting of separate packaging units, the kit further comprising at least one reference sample having a concentration of PlGF of > 15.6 ng/l, preferably of > 17.7 ng/l, particularly preferred of > 23.3 ng/l, and a concentration of sFlt-1 of < 56.5 ng/l, preferably of < 37.4 ng/l, and optionally further comprising an information means, according to which (i) a PlGF concentration in the upper two tertiles of a reference collective, and an sFlt-1 concentration in the lowest tertile of the reference collective, and/or (ii) a PlGF value above a PlGF reference value and an sFlt-1 value below a sFlt-1 reference value indicates an elevated probability for an adverse event, consisting of death, non-fatal myocardial infarction and/or stroke.

30. Use of a kit according to claim 29 for a diagnosis, risk stratification and/or monitoring of a vascular disease with atherosclerotic etiology, and/or for estimation of the probability of developing such a disease, or for carrying out the method according to any of claims 1 to 28.

31. Use of a diagnostic kit, comprising at least one means for quantifying PlGF and at least one means for quantifying sFlt-1 in a sample to be analyzed, optionally consisting of separate packaging units, the kit further comprising an information means, according to which (i) a PlGF concentration in the upper two tertiles of a reference collective, and an sFlt-l concentration in the lowest tertile of the reference collective, and/or (ii) a PlGF value above a PlGF reference value and an sFlt-1 value below a sFlt-1 reference value indicates an elevated probability for an adverse event, consisting of death, non-fatal myocardial infarction and/or stroke, for a diagnosis, risk stratification and/or monitoring of a vascular disease with atherosclerotic etiology, and/or for estimating the probability of developing such a disease, or for carrying out the method according to any of claims 1 to 28.

32. Use of an assay element, comprising a sample application zone for application of the sample and of labelled specific PlGF binding partners and sFlt-1 binding partners, wherein the binding partners are optionally present in the assay element, and wherein the sample application zone is contacting at least one detection zone, and the detection zone comprises spatially separated regions for specific binding of PlGF and sFlt-1, for a diagnosis, risk stratification and/or monitoring of a vascular disease with atherosclerotic etiology, and/or for estimating the probability of developing such a disease, and/or for the identification of a patient who is supposed to benefit from the treatment by one or more therapeutic agents, consisting of sFlt-1, anti-inflammatory agents, anti-thrombotics, anti-platelet agents, fibrinolytic agents, lipid lowering agents, direct thrombin inhibitors, and/or glycoprotein IIb/IIIa receptor inhibitors.

33. Use of an immunochromatic assay element comprising at lest one means for quantifying PlGF and at least one means for quantifying sFlt-1 in a sample to be analyzed for a diagnosis, risk stratification and/or monitoring of a vascular disease with atherosclerotic etiology, and/or for estimating the probability of developing such a disease, and/or for the identification of a patient who is supposed to benefit from the treatment by one or more therapeutic agents, consisting of sFlt-1, anti-inflammatory agents, anti-throbotics, anti-platelet agents, fibrinolytic agents, lipid lowering agents, direct thrombin inhibitors, and/or glykoprotein IIb/IIIa receptor inhibitors.

## Revendications

1. Utilisation d'un procédé *in vitro* comprenant les étapes suivantes :
(a) Mise à disposition d'un échantillon d'un patient à examiner ;
(b) Quantification de PlGF dans l'échantillon ;
(c) Quantification de sFlt-1 dans l'échantillon ;
à des, fins de diagnostic, stratification du risque et/ou surveillance d'une maladie vasculaire à étiologie athérosclérotique et/ou appréciation de la probabilité de développer une telle maladie.

2. Utilisation selon la revendication 1, le procédé comprenant, en outre, l'étape suivante :
(d) Comparaison des valeurs de PlGF et sFlt-1 déterminées en (b) et (c) respectivement avec une valeur de référence et/ou respectivement avec une valeur déterminée dans un échantillon de référence ;
et/ou les étapes suivantes :
(d') Etablissement d'un rapport entre la valeur de PlGF déterminée en (b) et la valeur de sFlt-1 déterminée en (c) ;
(e')Comparaison de la valeur déterminée en (d') avec une valeur de référence et/ou une valeur déterminée dans un échantillon de référence.

3. Utilisation selon la revendication 1 ou 2, dans laquelle la maladie vasculaire est une maladie cardiaque coronarienne, une maladie cérébrovasculaire et/ou une artériopathie oblitérante.

4. Utilisation selon la revendication 4, dans laquelle la maladie cardiaque coronarienne est un syndrome coronaire aigu, de préférence une angine de poitrine instable ou un infarctus du myocarde aigu.

5. Utilisation selon l'une des revendications précédentes, dans laquelle l'échantillon à examiner est du sang périphérique ou une fraction de celui-ci, de préférence du plasma sanguin ou sérum sanguin.

6. Utilisation selon l'une des revendications précédentes, dans laquelle la stratification du risque comprend une détermination d'une probabilité d'un événement défavorable, comprenant décès, infarctus du myocarde non mortel et/ou attaque.

7. Utilisation selon la revendication 6, dans laquelle une valeur de PlGF supérieure à une valeur de référence de PlGF de préférence > 15,6 ng/l, de façon particulièrement préférentielle > 17,7 ng/l, de façon tout particulièrement préférentielle > 23,3 ng/l, et une valeur de sFlt-1 inférieure à une valeur de référence de sFlt-1 de préférence < 56,5 ng/l, de façon particulièrement préférentielle < 37,4 ng/l, indique une probabilité accrue d'un événement défavorable.

8. Utilisation selon la revendication 6 ou 7, dans laquelle une concentration de PlGF se situant dans les deux tertiles supérieurs d'un groupe de référence, et une concentration de sFlt-1 se situant dans le tertile le plus inférieur du groupe de référence, indiquent une probabilité accrue d'un événement défavorable.

9. Utilisation selon l'une des revendications 6 à 8, dans laquelle un rapport de [PlGF = élevé : sFlt-1 = faible] indique une probabilité accrue d'un événement défavorable.

10. Utilisation selon la revendication 9, dans laquelle une concentration de PlGF > 15,6 ng/l, de préférence > 17,7 ng/l, de façon particulièrement préférentielle > 23,3 ng/l, signifie une concentration de PlGF élevée.

11. Utilisation selon la revendication 9 ou 10, dans laquelle une concentration de sFlt-1 < 56,5 ng/l, de préférence < 37,4 ng/l, signifie une concentration de sFlt-1 faible.

12. Utilisation selon l'une des revendications 9 à 11, dans laquelle un rapport de [PlGF : sFlt-1] ≥ 0,31, de préférence ≥ 0,42, de façon particulièrement préférentielle ≥ 0,62, indique une probabilité accrue d'un événement défavorable.

13. Utilisation selon l'une des revendications précédentes, dans laquelle le procédé comprend une quantification d'au moins un autre biomarqueur, de préférence VEGF, sCD40L, PAPP-A, MPO, myoglobine, créatine kinase, en particulier CK-MB, troponine, en particulier troponine I, troponine T et/ou leurs complexes, CRP, cystatine C, peptide natriurétique, en particulier ANB, BNP et/ou NT-proBNP.

14. Procédé *in vitro* pour diagnostic, stratification du risque et/ou surveillance d'une maladie vasculaire à étiologie athérosclérotique et/ou appréciation de la probabilité de développer une telle maladie, le procédé comprenant les étapes suivantes :
a) Mise à disposition d'un échantillon d'un patient à examiner ;
(b) Quantification de PlGF dans l'échantillon ;
(c) Quantification de sFlt-1 dans l'échantillon.

15. Procédé selon la revendication 14, le procédé comprenant, en outre, l'étape suivante :
(d) Comparaison des valeurs de PlGF et sFlt-1 déterminées en (b) et (c) respectivement avec une valeur de référence et/ou respectivement avec une valeur déterminée dans un échantillon de référence ;
et/ou les étapes suivantes :
(d') Etablissement d'un rapport entre la valeur de PlGF déterminée en (b) et la valeur de sFlt-1 déterminée en (c) ;
(e') Comparaison de la valeur déterminée en (d') avec une valeur de référence et/ou une valeur déterminée dans un échantillon de référence.

16. Procédé selon l'une des revendications 14 ou 15, dans lequel le patient présente une maladie vasculaire consistant en une maladie cardiaque coronarienne, une maladie cérébrovasculaire et/ou une artériopathie oblitérante, ou une suspicion de présenter ou de développer ladite maladie.

17. Procédé selon la revendication 16, dans lequel la maladie cardiaque coronarienne est un syndrome coronaire aigu, de préférence une angine de poitrine instable ou un infarctus du myocarde aigu.

18. Procédé selon l'une des revendications 14 à 17, dans lequel le patient est traité avec un ou plusieurs agents thérapeutiques, comprenant du sFlt-1, des agents anti-inflammatoires, des antithrombotiques, des agents actifs contre les plaquettes sanguines, des agents fibrinolytiques, des agents abaisseurs de lipides, des inhibiteurs directs de la thrombine et/ou des inhibiteurs des récepteurs à la glycoprotéine IIb/IIIa.

19. Procédé selon l'une des revendications 14 à 18, dans lequel l'échantillon à examiner est du sang périphérique ou une fraction de celui-ci, de préférence du plasma sanguin ou sérum sanguin.

20. Procédé selon l'une des revendications 14 à 19, dans lequel le procédé comprend, en outre, une stratification du risque par détermination d'une probabilité d'un événement défavorable, comprenant décès, infarctus du myocarde non mortel et/ou attaque.

21. Procédé selon la revendication 20, dans lequel une valeur de PlGF supérieure à une valeur de référence de PlGF de préférence > 15,6 ng/l, de façon particulièrement préférentielle > 17,7 ng/l, de façon tout particulièrement préférentielle > 23,3 ng/l, et une valeur de sFlt-1 inférieure à une valeur de référence de sFlt-1 de préférence < 56,5 ng/l, de façon particulièrement préférentielle < 37,4 ng/l, indique une probabilité accrue d'un événement défavorable.

22. Procédé selon la revendication 20 ou 21, dans lequel une concentration de PlGF se situant dans les deux tertiles supérieurs d'un groupe de référence, et une concentration de sFlt-1 se situant dans le tertile le plus inférieur du groupe de référence, indiquent une probabilité accrue d'un événement défavorable.

23. Procédé selon la revendication 20 à 22, dans lequel un rapport de [PlGF = élevé : sFlt-1 = faible] indique une probabilité accrue d'un événement défavorable.

24. Procédé selon la revendication 23, dans lequel une concentration de PlGF > 15,6 ng/l, de préférence > 17,7 ng/l, de façon particulièrement préférentielle > 23,3 ng/l, signifie une concentration de PlGF élevée.

25. Procédé selon la revendication 23 ou 24, dans lequel une concentration de sFlt-1 < 56,5 ng/l, de préférence < 37,4 ng/l, signifie une concentration de sFlt-1 faible.

26. Procédé selon l'une des revendications 23 à 25, dans lequel un rapport de [PlGF : sFlt-1] ≥ 0,31, de préférence ≥ 0,42, de façon particulièrement préférentielle ≥ 0,62, indique une probabilité accrue d'un événement défavorable.

27. Procédé selon l'une des revendications 14 à 26, le procédé comprenant une quantification d'au moins un autre biomarqueur, de préférence VEGF, sCD40L, PAPP-A, MPO, myoglobine, créatine kinase, en particulier CK-MB, troponine, en particulier troponine I, troponine T et/ou leurs complexes, CRP, cystatine C, peptide natriurétique, en particulier ANB, BNP et/ou NT-proBNP.

28. Utilisation d'un procédé selon l'une des revendications 14 à 27 pour identification d'un patient qui tirera vraisemblablement avantage du traitement utilisant un ou plusieurs agents thérapeutiques, comprenant du sFlt-1, des agents anti-inflammatoires, des antithrombotiques, des agents actifs contre les plaquettes sanguines, des agents fibrinolytiques, des agents abaisseurs de lipides, des inhibiteurs directs de la thrombine et/ou des inhibiteurs des récepteurs à la glycoprotéine IIb/IIIa.

29. Trousse de diagnostic, comprenant au moins un moyen de quantification de PlGF et au moins un moyen de quantification de sFlt-1 dans un échantillon à examiner, comprenant, au choix, des unités de conditionnement séparées, la trousse comprenant, en outre, au moins un échantillon de référence ayant une concentration de PlGF > 15,6 ng/l, de préférence > 17,7 ng/l, de façon particulièrement préférentielle > 23,3 ng/l, et une concentration de sFlt-1 < 56,5 ng/l, de préférence < 37,4 ng/l et, au choix, en outre, un moyen d'information, selon lequel (i) une concentration de PlGF, se situant dans les deux tertiles supérieurs d'un groupe de référence, et une concentration de Sflt-1 se situant dans le tertile le plus inférieur du groupe de référence, et/ou (ii) une valeur de PlGF supérieure à la valeur de référence de PlGF et une valeur de sFlt-1 inférieure à la valeur de référence de sFlt-1, indiquent une probabilité accrue d'un événement défavorable, comprenant décès, infarctus du myocarde non mortel et/ou attaque.

30. Utilisation d'une trousse selon la revendication 29 pour le diagnostic, la stratification du risque et/ou la surveillance d'une maladie vasculaire à étiologie athérosclérotique et/ou pour appréciation de la probabilité de développer une telle maladie, ou pour la réalisation du procédé selon l'une des revendications 1 à 28.

31. Utilisation d'une trousse de diagnostic, comprenant au moins un moyen de quantification de PlGF et au moins un moyen de quantification de sFlt-1 dans un échantillon à examiner, comprenant, au choix, des unités de conditionnement séparées, la trousse comprenant, en outre, un moyen d'information, selon lequel (i) une concentration de PlGF, se situant dans les deux tertiles supérieurs d'un groupe de référence, et une concentration de Sflt-1 se situant dans le tertile le plus inférieur du groupe de référence, et/ou (ii) une valeur de PlGF supérieure à la valeur de référence de PlGF et une valeur de sFlt-1 inférieure à la valeur de référence de sFlt-1 indique une probabilité accrue d'un événement défavorable, choisi dans le groupe comprenant décès, infarctus du myocarde non mortel et/ou attaque, pour le diagnostic, la stratification du risque et/ou la surveillance d'une maladie vasculaire à étiologie athérosclérotique et/ou pour appréciation de la probabilité de développer une telle maladie, ou pour la réalisation du procédé selon l'une des revendications 1 à 28.

32. Utilisation d'un élément de test, comprenant une zone d'application de l'échantillon pour application de l'échantillon et de partenaires de liaison de PlGF et partenaires de liaison de sFlt-1 spécifiques marqués, les partenaires de liaison étant présents, au choix, dans l'élément de test, et la zone d'application de l'échantillon étant en contact avec au moins une zone de détection et la zone de détection comprenant des zones séparées dans l'espace pour la liaison spécifique du PlGF et du sFTl-1, pour le diagnostic, la stratification du risque et/ou la surveillance d'une maladie vasculaire à étiologie athérosclérotique et/ou pour appréciation de la probabilité de développer une telle maladie, et/ou pour l'identification d'un patient qui tirera vraisemblablement avantage du traitement utilisant un ou plusieurs agents thérapeutiques, comprenant du sFlt-1, des agents anti-inflammatoires, des antithrombotiques, des agents actifs contre les plaquettes sanguines, des agents fibrinolytiques, des agents abaisseurs de lipides, des inhibiteurs directs de la thrombine et/ou des inhibiteurs des récepteurs à la glycoprotéine IIb/IIIa.

33. Utilisation d'un élément de test immunochromatique, comprenant au moins un moyen de quantification de PlGF et au moins un moyen de quantification de sFlt-1 dans un échantillon à examiner, pour le diagnostic, la stratification du risque et/ou la surveillance d'une maladie vasculaire à étiologie athérosclérotique et/ou pour appréciation de la probabilité de développer une telle maladie, et/ou pour l'identification d'un patient qui tirera vraisemblablement avantage du traitement utilisant un ou plusieurs moyens thérapeutiques, comprenant du sFlt-1, des agents anti-inflammatoires, des antithrombotiques, des agents actifs contre les plaquettes sanguines, des agents fibrinolytiques, des agents abaisseurs de lipides, des inhibiteurs directs de la thrombine et/ou des inhibiteurs des récepteurs à la glycoprotéine IIb/IIIa.
